# EUROPEAN PATENT SPECIFICATION

(11) **EP 1 396 542 B1**
(45) Date of publication and mention of the grant of the patent: **09.03.2011**
(21) Application number: 03025430.4
(22) Date of filing: 22.10.1993
(51) Int. Cl.: C12N 15/12, C07K 14/705, A61K 39/395

(54) **P-Selectin Ligand Protein**
P- Selectin Ligandenprotein
Protéine de ligand de p-sélectine

(30) Priority: 23.10.1992 US 965662; 26.08.1993 US 112608
(43) Date of publication of application: 10.03.2004
(62) Divisional of application: 94900380.0
(73) Proprietor: Genetics Institute, LLC, Cambridge, MA 02140 (US)
(72) Inventor: Larsen, Glenn R., Sudbury, MA 01776 (US); Sako, Dianne S., Boston, MA 02130 (US); Chang, Xiao-Jia, Lincoln, MA 01773 (US); Veldman, Geertruida M., Sudbury, MA 01776 (US)
(74) Representative: Dörries, Hans Ulrich

(56) References cited:
- WO-A-91/06632
- WO-A-92/01718
- WO-A-92/16612
- WO-A-94/11498
- MOORE K L ET AL: "IDENTIFICATION OF A SPECIFIC GLYCOPROTEIN LIGAND FOR P-SELECTIN (CD62) ON MYELOID CELLS" JOURNAL OF CELL BIOLOGY, ROCKEFELLER UNIVERSITY PRESS, NEW YORK, US, US, vol. 118, no. 2, July 1992 (1992-07), pages 445-456, XP001015345 ISSN: 0021-9525
- LARSEN G R ET AL: "P-SELECTIN AND E-SELECTIN. DISTINCT BUT OVERLAPPING LEUKOCYTE LIGAND SPECIFICITIES" JOURNAL OF BIOLOGICAL CHEMISTRY, AMERICAN SOCIETY OF BIOLOGICAL CHEMISTS, BALTIMORE, MD, US, vol. 267, no. 16, 5 June 1992 (1992-06-05), pages 11104-11110, XP002067822 ISSN: 0021-9258

## Description

### BACKGROUND OF THE INVENTION

The present invention relates to the field of anti-inflammatory substances which act by inhibiting leukocyte adhesion to endothelial cells. More particularly, the present invention is directed to a novel ligand for the mammalian adhesion protein known as "P-selectin."

During inflammation leukocytes adhere to the vascular endothelium and enter subendothelial tissue, an interaction which is mediated by specific binding of the selectin or LEC-CAM class of proteins to ligands on target cells. Such selectin-mediated cellular adhesion also occurs in thrombotic disorders and parasitic diseases and may be implicated in metastatic spread of tumor cells.

The selectin proteins are characterized by a N-terminal lectin-like domain, an epidermal growth factor-like domain, and regions of homology to complement binding proteins. Thus far three human selectin proteins have been identified, E-selectin (formerly ELAM-1), L-selectin (formerly LAM-1) and P-selectin (formerly PADGEM or GMP-140). E-selectin is induced on endothelial cells several hours after activation by cytokines, mediating the calcium-dependent interaction between neutrophils and the endothelium. L-selectin is the lymphocyte homing receptor, and P-selectin rapidly appears on the cell surface of platelets when they are activated, mediating calcium-dependent adhesion of neutrophils or monocytes to platelets. P-selectin is also found in the Weibel-Palade bodies of endothelial cells; upon its release from these vesicles P-selectin mediates early binding of neutrophils to histamine-or thrombin-stimulated endothelium.

Selectins are believed to mediate adhesion through specific interactions with ligands present on the surface of target cells. Generally the ligands of selectins are comprised at least in part of a carbohydrate moiety. For example, E-selectin binds to carbohydrates having the terminal structure 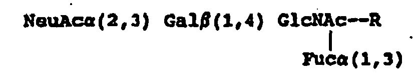 and also to carbohydrates having the terminal structure 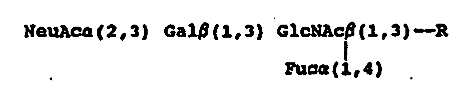 where R the remainder of the carbohydrate chain. These carbohydrates are known blood group antigens and are commonly referred to as sialylated Lewis^{x} and sialylated Lewis^{x}, respectively. The presence of the sialylated Lewis^{x} antigen alone on the surface of an endothelial cell may be sufficient to promote binding to an E-selectin expressing cell. E-selectin also binds to carbohydrates having the terminal structures

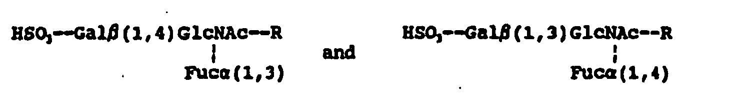

As with E-selectin, each selectin appears to bind to a range of carbohydrates with varying affinities. The strength of the selectin mediated adhesive event (binding affinity) may also depend on the density of the carbohydrate and on the density of the selectin on the cell surface.

P-selectin binds to carbohydrates containing the non-sialylated form of the Lewis^{x} blood group antigen and with higher affinity to sialylated Lewis^{x}. P-selectin may also recognize sulfatides, which are heterogeneous 3-sulfated galactosyl ceramides, isolated from myeloid and tumor cells by lipid extraction. However, the binding of cells bearing P-selectin to cells bearing P-salectin ligands is abolished when the ligand-bearing cells are treated with proteases, indicating that the P-selectin ligand may be a glycoprotein.

Two putative glycoprotein ligands for P-selectin have recently been identified, one of which has been partially purified, (Moore et al., J. Cell Biol. 118, 445-456 (1992)). However, neither amino acid composition nor the amino acid sequence of these glycoproteins are disclosed.

### SUMMARY OF THE IVENTION

In a first aspect, the present invention relates to a P-selectin ligand protein which is a fragment of the P-selectin ligand protein having P-selectin ligand activity comprising an amino acid sequence selected from:
a) amino acid 21 to amino acid 54 of SEQ ID NO:1;
b) amino acid 55 to amino acid 127 of SEQ ID NO:1;
c) amino acid 128 to amino acid 267 of SEQ ID NO:1:
d) amino acid 268 to amino acid 308 of SEQ ID NO:1;
e) amino acid 309 to amino acid 333 of SEQ ID NO:1;
f) amino acid 334 to amino acid 402 of SEQ ID NO:1;
g) amino acid 43 to amino acid 56 of SEQ ID NO:1;
h) amino acid 1 to amino acid310 of SEQ ID NO:1.

In a second aspect, the present invention concerns a nucleic acid molecule comprising a sequence encoding one of the P-selectin ligand fragments of the invention.

In a third aspect, the present invention relates to an antibody that specifically reacts with P-selectin ligand, wherein the sequence of the P-selectin ligand protein consists essentially of an amino acid sequence selected from:
a) amino acid 42 to amino acid 56 of SEQ ID NO:1; and
b) amino acid 127 to amino acid 138 of SEQ ID NO: 1.

In a fourth aspect, the present invention concerns a plasmid encoding a P-selectin ligand comprising a sequence according to SEQ ID NO:1.

In a fifth aspect, the present invention relates to the use of an antibody of the invention that specifically reacts with P-selectin-ligand protein for the preparation of a pharmaceutical composition for treating a condition characterized by P-selectin-medlated adhesion.

Likewise, the present invention relates to an antibody of the invention that specifically reacts with P-selectin ligand protein for use for the treatment or diagnosis of inflammatory diseases or cancer.

In a sixth aspect, the present invention relates to the use of an antibody of the invention that specifically reacts with P-selectin ligand protein for the preparation of a pharmaceutical composition for treating or diagnosing inflammatory diseases or cancer.

In a seventh aspect, the present invention relates to a fusion protein comprising
a) a first amino acid sequence chosen from: amino acid 21 to amino acid 54 of SEQ ID NO:1; amino acid 55 to amino acid 127 of SEQ ID NO:1; amino acid 128 to amino acid 267 of SEQ ID NO:1; amino acid 268 to amino acid 308 of SEQ ID NO:1; amino acid 309 to amino acid 333 of SEQ ID NO:1; amino acid 334 to amino acid 402 of SEQ ID NO:1; amino acid 43 to amino acid 56 of SEQ ID NO:1; and amino acid 42 to amino acid 295 of SEQ ID NO:1; and
b) a second amino acid sequence derived from the sequence of an Fc portion of an immunoglobulin.

In an eighth aspect, the present invention relates to a DNA encoding the fusion protein the invention.

In a ninth aspect, the present invention concerns the plasmid comprising a DNA encoding a soluble P-selectin ligand-Fc fusion protein.

### DETAILED DESCRIPTION OF THE INVENTION

The present inventors have for the first time identified and isolated a novel nucleic and comprising a sequence which encodes a protein which acts as a ligand for P-selectin on human endothelial cells and platelets. The complete amino acid sequence of the P-selectin ligand protein (i.e., the mature peptide plus the leader sequence) is characterized by the amino acid sequence set forth in SEQ ID NO:1 from amino acid 1 to amino acid 402. Hydrophobicity analysis and comparison with known cleavage patterns predict a signal sequence of 20 to 22 amino acids, i.e., amino acids 1 to 20 or amino acids 1 to 22 of SEQ ID NO:1. The P-selectin ligand protein contains a PACE (paired basic amino acid converting enzyme) cleavage site (-Arg-Asp-Arg-Arg-) at aminoacids 38-41 of SEQ ID NO:1. The mature P-selectin ligand protein is characterized by the amino acid sequence set forth in SEQ ID NO:1 from amino acid 42 to amino acid 402. A soluble form of the P-selectin ligand protein is characterized by containing amino acids 21 to 310 of SEQ ID NO:1. Another soluble form of the mature P-selectin ligand protein is characterized by the amino acid sequence set forth in SEQ ID NO:1 from amino acid 42 to amino acid 310. The soluble form of the P-selectin ligand protein is further characterized by being soluble in aqueous solution at room temperature. Of course, the corresponding DNA sequences as set forth in SEQ ID NO:1 encoding these proteins are also included in the subject invention.

The P-selectin ligand of the invention is a glycoprotein which may contain one or more of the following terminal carbohydrates: 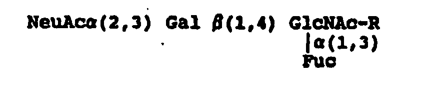 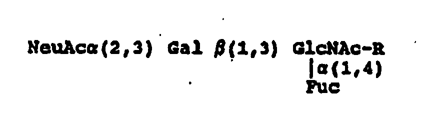 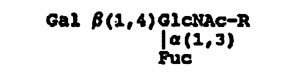 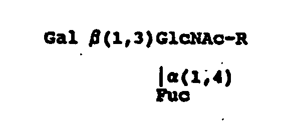 where R= the remainder of the carbohydrate chain, which is covalently attached either directly to the P-selectin ligand protein or to a lipid moiety which is covalently attached to the P-selectin ligand protein. The P-selectin ligand glycoprotein of the invention may additionally be sulfated or otherwise post-translationally modified. As expressed in COS and CHO cells, full length P-selectin ligand protein (amino acids 1 to 402 of SEQ ID NO:1 or amino acids 42 to 402 of SEQ ID NO:1) is a homodimeric protein having an apparent molecular of 220 kD as shown by non-reducing SDS-polyacrylamide gel electrophoresis.

Three regions of the P-selectin ligand protein of SEQ ID NO:1 are: an extracellular domain (from about amino acid 21 to 310 of SEQ ID NO:1), a transmembrane domain (from about amino acid 311 to 332 of SEQ ID NO:1), and an intracellular, cytoplasmic domain (from about amino acid 333 to 402 of SEQ ID NO:1). The extracellular domain contains three consensus tripeptide sites (Asn-X-Ser/Thr) of potential N-linked glycosylation beginning at Asn residues 65, 111, and 292. The extracellular domain further contains three potential sites of tyrosine sulfation at residues 46, 48, and 51. The region comprised of residues 55-267 contains a high percentage of proline, serine, and threonine including a subdomain of fifteen decamerio repeats of the ten amino acid consensus sequence Ala-Thr/Met-Glu-Ala-Gln-Thr-Thr-X-Pro/Leu-Ala/Thr, wherein X can be either Pro, Ala, Gln, Glu, or Arg. Regions such as these are characteristic of highly O-glycosylated proteins.

COS or CHO cells co-transfected with a gene encoding the P-selectin ligand protein and a gene encoding an (α1,3/1,4) fucosyltransferase (hereinafter 3/4FT) are capable of binding to CHO cells expressing P-selectin on their surface, but are not capable of binding to CHO cells which do not express P-selectin on their surface. In order to bind to P-selectin, either in purified form or expressed on the surface of CHO cells, the gene encoding the P-seleatin ligand protein must be co-transfected with the gene encoding a 3/4FT, since transfection of either gene in the absence of the other either abolishes or substantially reduces the P-selectin binding activity. The binding of the P-selectin ligand protein of the invention to P-selectin can be inhibited by EDTA or by a neutralizing monoclonal antibody specific for P-selectin. The binding of the P-selectin ligand protein of the invention to P-selectin is not inhibited by a non-neutralizing monoclonal antibody specific for P-selectin or by an isotype control. These results characterize the binding specificity of the P-selectin ligand protein of the invention.

For the purposes of the present invention, a protein is defined as having "P-selectin ligand protein activity", i.e., variably referred to herein as a "P-selectin ligand protein", or as a "P-selectin ligand glycoprotein" or simply as a "P-selectin ligand", when it binds in a calcium-dependent manner to P-selectin which is present on the surface of cells as in the CHO-P-selectin binding assay of Example 4, or to P-selectin which is affixed to another surface, for example, as the chimeric P-selectin-IgGγ1 protein of Example 4 is affixed to Petri dishes.

The glycosylation state of the P-selectin ligand protein of the invention was studied using a chimeric, soluble form of the P-selectin ligand protein, described in detail in Example 5(C) and designated sPSL.T7. The sPSL.T7 protein produced from COS cells co-transfected with 3/4FT is extensively modified by posttranslational glycosylation, as described in detail in Example 6(C). Thus, it is believed that both N- and O-linked oligosaccharide chains, at least some of which are sialylated, are present on the P-selectin ligand protein of the invention.

The P-selectin ligand protein of the invention may also bind to E-selectin. Conditioned medium from COS cells which have been co-transfected with the DNA encoding sPSL.T7 and with the DNA encoding 3/4FT, when coated on wells of plastic microtiter plates, causes CHO cells which express E-selectin to bind to the plates; however CHO cells which do not express E-selectin do not bind to such plates. The binding of CHO cells which express E-selectin to microtiter plates coated with conditioned medium from COS cells which have been co-transfected with the DNA encoding sPSL.T7 and with the DNA encoding 3/4FT is abolished in the presence of EDTA or of a neutralizing antibody specific for E-selectin. Conditioned medium from COS cells transfected only with the sPSL.T7 DNA does not cause binding of CHO cells which express E-selectin when coated on wells of microtiter plates. For these reasons, the P-selectin ligand protein of the invention is believed to be useful as an inhibitor of E-selectin-mediated intercellular adhesion in addition to P-selectin-mediated intercellular adhesion.

Fragments of the P-selectin ligand protein which are capable of interacting with P-selectin or which are capable or inhibiting P-selectin-mediated intercellular adhesion are the object of the present invention. Such fragments comprise amino acids 21 to 54 of SEQ ID NO:1, a region of the P-selectin ligand protein having a low frequency of serine and threonine residues; amino acids 55 to 127 of SEQ ID NO:1, having a high frequency of proline, serine, and threonine in addition to two consensus sequences for asparagine-linked glycosylation (Asn-X-Ser/Thr); another larger fragment, amino acids 128 to 267 of SEQ ID NO:1, having both a high frequency of proline, serine, and threonine and containing fifteen repeats of the following ten amino acid consensus sequence: Ala-(Thr/Met)-Glu-Ala-GIn-Thr-Thr-(Pro/Arg/Gln/Ala/Glu)-(Leu/Pro)-(Ala/Thr) (smaller fragments within this large fragment may also retain the capacity to interact with P-selectin or act as inhibitors of P-selectin-mediated intercellular adhesion); the region containing a consensus sequence for asparagine-linked glycosylation and comprising amino acids 268 to 308 of SEQ ID NO:1; the hydrophobic region of the protein represented by amino acids 309 to 333 of SEQ ID NO:1; and the amphiphilic region of the P-selectin ligand protein from amino acids 334 to 402. Additional fragments may comprise amino acid 43 to amino acid 56 of SEQ ID NO:1, with one or more sulfated tyrosines at amino acid 46, amino acid 48, and/or amino acid 51. Fragments of the P-selectin ligand protein may be in linear form or they may be cyclized using known methods, for example, as described in H.U. Saragovi, et al., Bio/Technology 10, 773-778 (1992) and in R.S. McDowell, et al., J. Amer. Chem. Soc. 114. 9245-9253 (1992). For the purposes of the present invention, all references to "P-selectin ligand protein" herein include fragments capable of binding to P-selectin. These fragments are the object of the invention.

Such fragments may be fused to carrier molecules such as immunoglobulins, to increase the valency of P-selectin ligand of binding sites. For example, soluble forms of the P-selectin ligand protein such as the fragment from amino acid 42 to amino acid 295 of SEQ ID NO:1 may be fused through "linker" sequences to the Fc portion of an immunoglobulin. For a bivalent form of the P-selectin ligand protein, such a fusion could be to the Fc portion of an IgG molecule as in Example 5(D) and in SEQ ID NO:6. Other immunoglobulin isotypes may also be used to generate such fusions. For example, a P-selectin ligand protein - IgM fusion would generate a decavalent form of the P-selectin ligand protein of the invention.

As detailed in the Examples below, the P-selectin ligand protein was initially obtained using an expression cloning approach (Clark et al. U.S. 4,675,285). A cDNA library was constructed from the human promyelocytic cell line HL60 (S.J. Collins, et al., Nature 270, 347-349 (1977), ATCC No. CCL 240) . This library was cotransfected into COS cells with a DNA encoding a 3/4 FT, and the cotransfectants were screened for binding to a chimeric molecule consisting of the extracellular portion of P-selectin and the Fc portion of a human IgGγ1 monoclonal antibody. Cotransfectants which bound to the chimeric P-selectin were enriched for cDNAs encoding the P-selectin ligand protein. This screening process was repeated several times to enrich the plasmid population further for cDNAs encoding the P-selectin ligand protein. In a second cloning stage, the enriched plasmid population was again cotransfected into COS cells with the 3/4FT gene and screened for binding to a fluorescently labeled CHO cell line which expressed P-selectin on the cell surface. A single cDNA clone was obtained from this approach and was designated pMT21:PL85. The pMT21:PL85 plasmid was deposited with the American Type Culture Collection on October 16, 1992 and given the accession number ATCC 69096.

One novel DNA is set forth in SEQ ID NO:1. This DNA may encode a variety of forms of the P-selectin ligand protein. For example, it encodes the entire P-selectin ligand protein having the amino acid sequence set forth in SEQ ID NO:1 from amino acid 1 to amino acid 402. In another embodiment, it encodes a form of the P-selectin ligand protein which lacks the signal sequence and which is characterized by the amino acid sequence set forth in SEQ ID NO:1 from amino acid 21 to amino acid 402. It also encodes the mature P-selectin ligand protein characterized by the amino acid sequence set forth in SEQ ID NO:1 from amino acid 42 to amino acid 402. This DNA is also embodied in a DNA sequence encoding a soluble form of the mature P-selectin ligand protein, said protein being characterized by the amino acid sequence set forth in SEQ ID NO:1 from amino acid 42 to amino acid 310. This DNA is further embodied in a DNA sequence encoding a soluble form of the P-selectin ligand protein which lacks the signal sequence, said protein being characterized by the amino acid sequence set forth in SEQ ID NO:1 from amino acid 21 to amino acid 310. In one embodiment, the present invention encodes a soluble form of the P-selectin ligand protein characterized by the amino acid sequence set forth in SEQ ID NO:1 from amino acid 1 to amino acid 310. The DNA of the present invention is free from association with other human DNAs and is thus characterized as an isolated DNA. As detailed above, DNAs which encode P-selectin ligand fragments which interact with P-selectin are the object of the present invention.

The expression of P-selectin ligand protein mRNA transcripts has been observed in a variety of human cell lines (HL-60, THP-1, U937) and in human monocytes and polymorphonuclear leukocytes by Northern analysis using a P-selectin ligand protein cDNA probe. In all of these cell lines, a major transcript of 2.5 kb was observed. A minor species of approximately 4 kb was observed in the HL60 and U937 cell lines and in polymorphonuclear leukocytes. In contrast, no P-selectin ligand mRNA expression was detected in the human hepatoblastoma cell line HepG2.

The P-selectin ligand protein referred to herein is encoded by a single copy gene and is not part of a multi-gene family, as determined by Southern blot analysis. The genomic form of the P-selectin ligand protein contains a large intron of approximately 9 kb located at nucleotide 54 in the 5' untranslated region. In polymorphonuclear leukocytes and monocytes, the P-selectin ligand protein is encoded by the DNA sequence set forth in SEQ ID NO:3 and contains sixteen repeat regions.

For the purposes of the present invention all references herein to the "DNA of SEQ ID NO:1" include, in addition to the specific DNA sequence set forth in SEQ ID NO:1, DNA sequences encoding the mature P-selectin ligand protein of SEQ ID NO:1; DNA sequences encoding fragments of the . P-selectin ligand protein of SEQ ID NO:1 which are capable of binding to P-selectin; DNA sequences encoding soluble forms of the P-selectin ligand protein of SEQ ID NO:1; allelic variations of the DNA sequence of SEQ ID NO:1; DNAs which hybridize to the DNA sequence of SEQ ID NO:1 and which encode proteins having P-selectin ligand protein activity; DNAs which differ from the DNA of SEQ ID NO:1 by virtue of degeneracy of the genetic code; and the variations of the DNA sequence of SEQ ID NO:1 set forth above. Similarly, all references to the "DNA of SEQ ID NO:3" include in addition to the specific DNA sequence set forth in SEQ ID NO:3, DNA sequences encoding the mature P-selectin ligand protein of SEQ ID NO:3; DNA sequences encoding fragments of the P-selectin ligand protein of SEQ ID NO:3 which are capable of binding to P-selectin; DNA sequences encoding soluble forms of the P-selectin ligand protein of SEQ ID NO:3; allelic variations of the DNA sequence of SEQ ID NO:3; DNAs which hybridize to the DNA sequence of SEQ ID NO:3 and which encode proteins having P-selectin ligand protein activity; DNAs which differ from the DNA of SEQ ID NO:3 by virtue of degeneracy of the genetic code; and the variations of the DNA sequence of SEQ ID NO:3 set forth above.

A DNA encoding a soluble form of the P-selectin ligand protein may be prepared by expression of a modified DNA in which the regions encoding the transmembrane and cytoplasmic domains of the P-selectin ligand protein are deleted and/or a stop codon is introduced 3' to the codon for the amino acid at the carboxy terminus of the extracellular domain. For example, hydrophobicity analysis predicts that the P-selectin ligand protein set forth in SEQ ID NO:1 has a transmembrane domain comprised of amino acids 311 to 332 of SEQ ID NO:1 and a cytoplasmic domain comprised of amino acids 333 to 402 of SEQ ID NO:1. A modified DNA as described above may be made by standard molecular biology techniques, including site-directed mutagenesis methods which are known in the art or by the polymerase chain reaction using appropriate oligonucleotide primers. Methods for producing several DNAs encoding various soluble P-selectin ligand proteins are set forth in Example 5.

The isolated DNA described herein may be operably linked to an expression control sequence such as the pMT2 or pED expression vectors disclosed in Kaufman et al., Nucleic Acids Res. 19, 4485-4490 (1991), in order to produce the P-selectin ligand recombinantly. Many suitable expression control sequences are known in the art. General methods of expressing recombinant proteins are also known and are exemplified in R. Kaufman, Methods in Enzymology 185, 537-566 (1990). As defined herein "operably linked" means enzymatically or chemically ligated to form a covalent bond between the isolated DNA of the invention and the expression control sequence, in such a way that the P-selectin ligand protein is expressed by a host cell which has been transformed (transfected) with the ligated DNA/expression control sequence.

Several endoproteolytic enzymes are known which cleave precursor peptides at the carboxyl side of paired amino acid sequences (e.g., -Lys-Arg- and -Arg-Arg-) to yield mature proteins. Such enzymes are generally known as paired basic amino acid converting enzymes or PACE, and their use in recombinant production of mature peptides is extensively disclosed in WO 92/09698 and U.S. Application Serial No. 07/885,972. The PACE family of enzymes are known to increase the efficiency of proteolytic processing of precursor polypeptides in recombinant host cells. As mentioned above, the fragments of the P-selectin ligand protein of the invention contain such a PACE cleavage site.

The soluble mature P-selectin ligand protein of the present invention may be made by a host cell which contains a DNA sequence encoding any soluble P-selectin ligand protein as described herein and a DNA sequence encoding PACE as described in WO 92/09698 and U.S. Application Serial No. 07/885,972, or using the DNA sequence of SEQ ID NO:5. Such a host cell may contain the DNAs as the result of co-transformation or sequential transformation of separate expression vectors containing the soluble P-selectin ligand protein DNA and the PACE DNA, respectively. A third DNA which encodes a 3/4FT may also be co-transformed with the DNAs encoding the P-selectin ligand protein and PACE. Alternatively, the host cell may contain the DNAs as the result of transformation of a single expression vector containing both soluble P-selectin ligand protein DNA and PACE DNA. Construction of such expression vectors is within the level of ordinary skill in molecular biology. Methods for co-transformation and transformation are also known.

Many DNA sequences encoding PACE are known. For example, a DNA encoding one form of PACE, known as furin, is disclosed in A.M.W. van den Ouweland et al., Nucl. Acids Res. 18, 664 (1990). A cDNA encoding a soluble form of PACE, known as PACESOL, is set forth in SEQ ID NO:5. DNAs encoding other forms of PACE also exist, and any such PACE-encoding DNA may be used to produce the soluble mature P-selectin ligand protein of the invention, so long as the PACE is capable of cleaving the P-selectin ligand protein at amino acids 38-41. Preferably, a DNA encoding a soluble form of PACE is used to produce the soluble mature P-selectin ligand protein of the present invention.

The DNAs encoding a soluble form of the P-selectin ligand protein and PACE, separately or together, may be operably linked to an expression control sequence such as those contained in the pMT2 or pED expression vectors discussed above, in order to produce the PACE-cleaved soluble P-selectin ligand recombinantly. Additional suitable expression control sequences are known in the art. Examples 3(C) and 3(D) below set forth methods for producing the soluble mature P-selectin ligand protein of the invention.

A number of types of cells may act as suitable host cells for expression of the P-selectin ligand protein. Suitable host cells are capable of attaching carbohydrate side chains characteristic of functional P-selectin ligand protein. Such capability may arise by virtue of the presence of a suitable glycosylating enzyme within the host cell, whether naturally occurring, induced by chemical mutagenesis, or through transfection of the host cell with a suitable expression plasmid containing a DNA sequence encoding the glycosylating enzyme. Host cells include, for example, monkey COS cells, Chinese Hamster Ovary (CHO) cells, human kidney 293 cells, human epidermal A431 cells, human Colo205 cells, 3T3 cells, CV-1 cells, other transformed primate cell lines, normal diploid cells, cell strains derived from in vitro culture of primary tissue, primary explanta, HeLa cells, mouse L cells, BHK, HL-60, U937, or Hak cells.

The P-selectin ligand protein may also be produced by operably linking the isolated DNA of the invention and one or more DNAs encoding suitable glycosylating enzymes to suitable control sequences in one or more insect expression vectors, and employing an insect expression system. Materials and methods for baculovirus/insect cell expression systems are commercially available in kit form from, *e.g*., Invitrogen, San Diego, California, U.S.A. (the MaxBace kit), and such methods are well known in the art, as described in summers and Smith, Texas Agricultural Experiment Station Bulletin No. 1555 (1987). Soluble forms of the P-selectin ligand protein may also be produced in insect cells using appropriate isolated DNAs as described above. A DNA encoding a form of PACE may further be co-expressed in an insect host cell to produce a PACE-cleaved form of the P-selectin ligand protein.

Alternatively, it may be possible to produce the P-selectin ligand protein in lower eukaryotes such as yeast or in prokaryotes such as bacteria. Potentially suitable yeast strains include *Saccharomyces cerevisiae,* S*chizosaccharomyces pombe*, *Kluyveromyces* strains, *Candida,* or any yeast strain capable of expressing heterologous proteins. Potentially suitable bacterial strains include *Escherichia coli, Bacillus subtilis, Salmonella typhimurium*, or any bacterial strain capable of expressing heterologous proteins. If the P-selectin ligand protein is made in yeast or bacteria, it is necessary to attach the appropriate carbohydrates to the appropriate sites on the protein moiety covalently, in order to obtain the glycosylated P-selectin ligand protein. Such covalent attachments may be accomplished using known chemical or enzymatic methods.

The fragments of the P-selectin ligand protein of the invention may also be expressed as a product of transgenic animals, e.g., as a component of the milk of transgenic cows, goats, pigs, or sheep which are characterized by somatic or germ cells containing a DNA sequence encoding the P-selectin ligand protein.

The fragments of the P-selectin ligand protein of the invention nay be prepared by culturing transformed host cells under culture conditions necessary to express a P-selectin binding glycoprotein. The resulting expressed glycoprotein may then be purified from culture medium or cell extracts. Soluble forms of the P-selectin ligand protein of the invention can be purified by affinity chromatography over Lentil lectin-Sepharose® and subsequent elution with 0.5M α-methyl-mannoside. The eluted soluble P-selectin ligand protein can then be further purified and concentrated by a 0-70% ammonium sulfate precipitation step. The protein is then recovered, resuspended, and further purified by size exclusion chromatography over a TSK G4000SW_{XL}. Alternatively, full length forms of the P-selectin ligand protein of the invention can be purified by preparing a total membrane fraction from the expressing cell and extracting the membranes with a non-ionic detergent such as Triton X-100. The detergent extract can then be passed over an affinity column comprised of immobilized P-selectin, and the P-selectin ligand protein can be eluted from the column with 10mM EDTA in a buffer containing 0.1% detergent. The material eluted from the affinity column can then be dialyzed to remove EDTA and further purified over a Lentil lectin-Sepharose® affinity column, again eluting with 0.5M α-mothyl-mannoside.

Alternatively, the fragments of the P-selectin ligand protein of the invention are concentrated using a commercially available protein concentration filter, for example, an Amicon or Millipore Pellicon ultrafiltration unit. Following the concentration step, the concentrate can be applied to a purification matrix such as a gel filtration medium. Alternatively, an anion exchange resin can be employed, for example, a matrix or substrate having pendant diethylaminoethyl (DEAE) groups. The matrices can be aorylamide, agarose, dextran, cellulose or other types commonly employed in protein purification. Alternatively, a cation exchange step can be employed. Suitable cation exchangers include various insoluble matrices comprising sulfopropyl or carboxymethyl groups. Sulfopropyl groups are preferred (e.g., S-Sepharose® columns). The purification of the P-selectin ligand protein from culture supernatant may also include one or more column steps over such affinity resins as concanavalin A-agarose, heparin-toyopearl® or Cibacrom blue 3GA Sepharose® ; or by hydrophobic interaction chromatography using such resins as phenyl ether, butyl ether, or propyl ether; or by immunoaffinity chromatography.

Finally, one or more reverse-phase high performance liquid chromatography (RP-HPLC) steps employing hydrophobic RP-HPLC media, e.g., silica gel having pendant methyl or other aliphatic groups, can be employed to further purify the P-selectin ligand protein. Some or all of the foregoing purification steps, in various combinations, can also be employed to provide a substantially homogeneous isolated recombinant protein. The P-selectin ligand protein thus purified is substantially free of other mammalian proteins and is defined in accordance with the present invention as "isolated P-selectin ligand protein".

The isolated P-selectin ligand protein may be useful in treating conditions characterized by P-selectin mediated intercellular adhesion. Such conditions include, without limitation, myocardial infarction, bacterial or viral infection, metastatic conditions, inflammatory disorders such as arthritis, acute respiratory distress syndrome, asthma, emphysema, delayed type hypersensitivity reaction, systemic lupus erythematosus, thermal injury such as burns or frostbite, autoimmune thyroiditis, experimental allergic encephalomyelitis, multiple sclerosis, multiple organ injury syndrome secondary to trauma, diabetes, Reynaud's syndrome, neutrophilic dermatosis (sweet's syndrome), inflammatory bowel disease, Grave's disease, glomerulonephritis, gingivitis, periodontitis, hemolytic uremic syndrome, ulcerative colitis, Crohn's disease, necrotizing enterocolitis, granulocyte transfusion associated syndrome, cytokine-induced toxicity, and the like. The isolated P-selectin ligand protein may also be useful in organ transplantation, both to prepare organs for transplantation and to quell organ transplant rejection. The isolated P-selectin ligand protein may be used to treat hemodialysis and leukophoresis patients. Additionally, the isolated P-selectin ligand protein may be used as an antimetastatic agent. The isolated P-selectin ligand protein may be used itself as an inhibitor of P-selectin-mediated intercellular adhesion or to design inhibitors of P-selectin-mediated intercellular adhesion. The present invention encompasses both pharmaceutical compositions containing isolated P-selectin ligand protein and therapeutic uses which employ the isolated P-selectin ligand protein.

The isolated P-selectin ligand protein, purified from cells or recombinantly produced, may be used as a pharmaceutical composition when combined with a pharmaceutically acceptable carrier. Such a composition may contain, in addition to the P-selectin ligand protein and carrier, diluents, fillers, salts, buffers, stabilizers, solubilizers, and other materials well known in the art. The term "pharmaceutically acceptable" means a non-toxic material that does not interfere with the effectiveness of the biological activity of the active ingredient(s). The characteristics of the carrier will depend on the route of administration. The pharmaceutical composition of the invention may also contain cytokines, lymphokines, or other hematopoietic factors such as M-CSF, GM-CSF, IL-1, IL-2, IL-3, IL-4, IL-5, IL-6, IL-7, IL-8, IL-9, IL-10, IL-11, IL-12, G-CSF, Meg-CSF, stem cell factor, and erythropoietin. The pharmaceutical composition may contain thrombolytic or anti-thrombotic factors such as plasminogen activator and Factor VIII. The pharmaceutical composition may further contain other anti-inflammatory agents. Such additional factors and/or agents may be included in the pharmaceutical composition to produce a synergistic effect with the isolated P-selectin ligand protein, or to minimize side effects caused by the isolated P-selectin ligand protein. Conversely, the isolated P-selectin ligand protein may be included in formulations of the particular cytokine, lymphokine, other hematopoietic factor, thrombolytic or anti-thrombotic factor, or anti-inflammatory agent to minimize side effects of the cytokine, lymphokine, other hematopoietic factor, thrombolytic or anti-thrombotic factor, or anti-inflammatory agent.

The pharmaceutical composition of the invention may be in the form of a liposome in which the isolated P-selectin ligand protein is combined, in addition to other pharmaceutically acceptable carriers, with amphipathic agents such as lipids which exist in aggregated form as micelles, insoluble monolayers, liquid crystals, or lamellar layers which in aqueous solution. Suitable lipids for liposomal formulation include, without limitation, monoglycerides, diglycerides, sulfatides, lysolecithin, phospholipids, saponin, bile acids, and the like. Preparation of such liposomal formulations is within the level of skill in the art, as disclosed, for example, in U.S. Patent No. 4,235,871; U.S. Patent No. 4,501,728; U.S. Patent No. 4,837,028; and U.S. Patent No. 4,737,323.

As used herein, the term "therapeutically effective amount" means the total amount of each active component of the pharmaceutical composition or method that is sufficient to show a meaningful patient benefit, i.e., healing of chronic conditions characterized by P-selectin-mediated cellular adhesion or increase in rate of healing of such conditions. When applied to an individual active ingredient, administered alone, the term refers to that ingredient alone. When applied to a combination, the term refers to combined amounts of the active ingredients that result in the therapeutic effect, whether administered in combination, serially or simultaneously.

In practicing the use of the present invention, a therapeutically effective amount of isolated P-selectin ligand protein is administered to a mammal having a P-salectin-mediated disease state. The isolated P-selectin ligand protein may be administered in accordance with the use of the invention either alone or in combination with other therapies such as treatments employing cytokines, lymphokines or other hematopoietic factors. When co-administered with one or more cytokines, lymphokines or other hematopoietic factors, the isolated P-selectin ligand protein may be administered either simultaneously with the cytokine(s), lymphokine(s), other hematopoietic factor(s), thrombolytic or anti-thrombotic factors, or sequentially. If administered sequentially, the attending physician will decide on the appropriate sequence of administering the isolated P-selectin ligand protein in combination with cytokine(s), lymphokine(s), other hematopoietic factor(s), thrombolytic or anti-thrombotic factors.

Administration of the isolated P-selectin ligand protein used in the pharmaceutical composition or to practice the use of the present invention can be carried out in a variety of conventional ways, such as oral ingestion, inhalation, or cutaneous, subcutaneous, or intravenous injection. Intravenous administration to the patient is preferred.

When a therapeutically effective amount of isolated P-selectin ligand protein is administered orally, the isolated P-selectin ligand protein will be in the form of a tablet, capsule, powder, solution or elixir. When administered in tablet form, the pharmaceutical composition of the invention may additionally contain a solid carrier such as a gelatin or an adjuvant. The tablet, capsule, and powder contain from about 5 to 95% isolated P-selectin ligand protein, and preferably from about 25 to 90% isolated P-selectin ligand protein. When administered in liquid form, a liquid carrier such as water, petroleum, oils of animal or plant origin such as peanut oil, mineral oil, soybean oil, or sesame oil, or synthetic oils may be added. The liquid form of the pharmaceutical composition may further contain physiological saline solution, dextrose or other saccharide solution, or glycols such as ethylene glycol, propylene glycol or polyethylene glycol. When administered in liquid form, the pharmaceutical composition contains from about 0.5 to 90% by weight of the isolated P-selectin ligand protein and preferably from about 1 to 50% isolated P-selectin ligand protein.

When a therapeutically effective amount of isolated P-selectin ligand protein is administered by intravenous, cutaneous or subcutaneous injection, the isolated P-selectin ligand protein will be in the form of a pyrogen-free, parenterally acceptable aqueous solution. The preparation of such parenterally acceptable protein solutions, having due regard to pH, isotonicity, stability, and the like, is within the skill in the art. A preferred pharmaceutical composition for intravenous, cutaneous, or subcutaneous injection should contain, in addition to the isolated P-selectin ligand protein an isotonio vehicle such as Sodium Chloride Injection, Ringer's Injection,Dextrose Injection, Dextrose and Sodium Chloride Injection, Lactated Ringer's Injection, or other vehicle as known in the art. The pharmaceutical composition of the present invention may also contain stabilizers, preservatives, buffers, antioxidants, or other additive known to those of skill in the art.

The amount of isolated P-selectin ligand protein in the pharmaceutical composition of the present invention will depend upon the nature and severity of the condition being treated, and on the nature of prior treatments which the patient has undergone. Ultimately, the attending physician will decide the amount of isolated P-selectin ligand protein with which to treat each individual patient. Initially, the attending physician will administer low doses of the isolated P-selectin ligand protein and observe the patient's response. Larger doses of isolated P-selectin ligand protein may be administered until the optimal therapeutic effect is obtained for the patient, and at that point the dosage is not increased further. It is contemplated that the various pharmaceutical compositions used to practice the method of the present invention should contain about 0.1 µg to about 100 mg of isolated P-selectin ligand protein per kg body weight.

The duration of intravenous therapy using the pharmaceutical composition of the present invention will vary, depending on the severity of the disease being treated and the condition and potential idiosyncratic response of each individual patient. It is contemplated that the duration of each application of the isolated P-selectin ligand protein will be in the range of 12 to 24 hours of continuous intravenous administration. Ultimately the attending physician will decide on the appropriate duration of intravenous therapy using the pharmaceutical composition of the present invention.

The isolated P-selectin ligand protein of the invention may also be used to immunize animals to obtain polyclonal and monoclonal antibodies which specifically react with the P-selectin ligand protein and which may inhibit p-selectin-madiated cellular adhesion. Such antibodies may be obtained using the entire P-selectin ligand protein as an immunogen, or by using fragments of the P-selectin ligand protein such as the soluble mature P-selectin ligand protein. Smaller fragments of the P-selectin ligand protein may also be used to immunize animals, such as the fragments set forth below: amino acid 42 to amino acid 56 of SEQ ID NO:1 and amino acid 127 to amino acid 138 of SEQ ID NO:1. An additional peptide immunogen comprises amino acid 238 to amino acid 248 of SEQ ID NO:1, with an alanine residue added to the amino terminus of the peptide. Another peptide immunogen comprises amino acid 43 to amino acid 56 of SEQ ID NO:1 having a sulfated tyrosine in any or all of positions 46, 48 or 51. The peptide immunogens additionally may contain a cysteine residue at the carboxyl terminus, and are conjugated to a hapten such as keyhole limpet hemocyanin (KLH). Additional peptide immunogens may be generated by replacing tyrosine residues with sulfated tyrosine residues. Methods for synthesizing such peptides are known in the art, for example, as in R.P. Merrifield, J.Amer.Chem.Soc. 85, 2149-2154 (1963); J.L. Krstenansky, et al., FEBS Lett. 211, 10 (1987).

Monoclonal antibodies binding to the P-selectin ligand glycoprotein or to complex carbohydrate moieties characteristic of the P-selectin ligand glycoprotein may be useful diagnostic agents for the immunodetection of inflammatory diseases and some forms of cancer. Some cancerous cells, such as small cell lung carcinomas, may express detectable levels of the P-selectin ligand protein. This abnormal expression of the P-selectin ligand protein by cancer cells may play a role in the metastasis of these cells.

Neutralizing monoclonal antibodies binding to the P-selectin ligand glycoprotein or to complex carbohydrates characteristic of the P-selectin ligand glycoprotein may also be useful therapeutics for both inflammatory diseases and also in the treatment of some forms of cancer where abnormal expression of the P-selectin ligand protein is involved. These neutralizing monoclonal antibodies are capable of blocking the selectin mediated intercellular adherence function of the P-selectin ligand protein. By blocking the binding of the P-selectin ligand protein, the adherence of leukocytes to sites of inappropriate inflammation is either abolished or markedly reduced. In the case of cancerous cells or leukemic cells, neutralizing monoclonal antibodies against the P-selectin ligand protein may be useful in detecting and preventing the metastatic spread of the cancerous cells which may be mediated by the P-selectin ligand protein. In addition, the monoclonal antibodies bound to these cells may target the cancerous cells for antibody-dependent cell medicated cytoxicity (ADCC), thus helping to eliminate the cancerous cells. Human antibodies which react with the P-selectin ligand protein may be produced in transgenic animals which contain human immunoglobulin encoding genes in their germ lines. Example 7 below sets forth production of a rabbit polyclonal antibody specific for P-selectin ligand protein fragments.

The P-selectin ligand protein of the invention may also be used to screen for agents which are capable of binding to the P-selectin ligand protein and thus may act as inhibitors of P-selectin mediated intercellular adhesion. Binding assays using a desired binding protein, immobilized or not, are well known in the art and may be used for this purpose using the P-selectin ligand protein of the invention. Appropriate screening assays may be cell-based, as in Example 3 below. Alternatively, purified protein based screening assays may be used to identify such agents. For example, P-selectin ligand protein may be immobilized in purified form on a carrier and binding to purified P-selectin may be measured in the presence and in the absence of potential inhibiting agents. A suitable binding assay may alternatively employ purified P-selectin immobilized on a carrier, with a soluble form of the P-selectin ligand protein of the invention.

Any P-selectin ligand protein may be used in the screening assays described above. For example, the full-length P-selectin ligand protein set forth in SEQ ID NO:1 from amino acid 1 to amino acid 402 may be used to screen for inhibitors; or the mature P-selectin ligand protein set forth in SEQ ID NO:1 from amino acid 42 to amino acid 402 may be used to screen for inhibitors, or the soluble mature P-selectin ligand protein set forth in SEQ ID NO:1 from amino acid 42 to amino acid 310 may be used to screen for inhibitors. Alternatively, the P-selectin ligand protein of SEQ ID NO:3 from amino acid 1 to amino acid 412, or a mature form of the P-selectin ligand protein as set forth in SEQ ID NO:3 from amino acid 42 to amino acid 412, or a soluble mature form of the P-selectin ligand protein set forth in SEQ ID NO:3 from amino acid 42 to amino acid 320 may be used to screen for inhibitors of intercellular adhesion in accordance with the present invention.

In such a screening assay, a first binding mixture is formed by combining P-selectin and the P-selectin ligand protein, and the amount of binding in the first binding mixture (Bₒ.) is measured. A second binding mixture is also formed by combining P-selectin, the P-selectin ligand protein, and the compound or agent to be screened, and the amount of binding in the second binding mixture (B) is measured. The amounts of binding in the first and second binding mixtures are compared, for example, by performing a B/Bₒ. calculation. A compound or agent is considered to be capable of inhibiting P-selectin mediated intercellular adhesion if a decrease in binding in the second binding mixture as compared to the first binding mixture is observed. The formulation and optimization of binding mixtures is within the level of skill in the art, such binding mixtures may also contain buffers and salts necessary to enhance or to optimize binding, and additional control assays may be included in the screening assay of the invention.

Compounds found to reduce by at least about 10%, preferably greater than about 50% or more of the binding activity of P-selectin ligand protein to P-selectin may thus be identified and then secondarily screened in other selectin binding assays, including assays of binding to E-selectin and to L-selectin and *in vivo* assays. By these means compounds having inhibitory activity for salectin-mediated intercellular adhesion which may be suitable as anti-inflammatory agents may be identified.

### EXAMPLE 1

### CLONING OF THE P-SELECTIN LIGAND PROTEIN GENE

### A. Construction of the HL60 cDNA library

An HL60 cDNA library was constructed for expression cloning the P-selectin ligand. PolyA⁺ RNA was isolated from total RNA from the human promyelocytic cell line HL60 (S.J. Collins, et al., *supra*) using a Fast Track mRNA Isolation Kit (Invitrogen; San Diego, CA). Double stranded cDNA was synthesized from the polyA⁺ RNA fraction and blunt-end ligated with EcoRI adaptors (5'-AATTCCGTCGACTCTAGAG-3',5' CTCTAGAGTCGACGG-3'). The cDNA was ligated into the expression vector pMT21 (R. Kaufman et al., J. Mol. Cell. Biol. 9, 946-958 (1989) that had been incubated sequentially with EcoRI endonuclease and calf intestinal alkaline phosphatase and gel purified. The ligation product was electroporated in 2 µl aliquots into competent E. coli DH5α cells and grown in 1 ml of SOB medium (J. Sambrook et al., Molecular Cloning: A Laboratory Manual, New York, Cold Spring Harbor Laboratory Press, pl.90 (1989)) which has been supplemented with 10 mM MgCl₂, 10 mM MgSO₄, and 2 % glycerol for one hour at 37°C. In order to divide the library into smaller subsets, an aliquot from each ml of bacterial suspension was plated onto agar plates in the presence of ampicillin, and the number of colonies per ml was calculated. Assuming that each colony represented one cDNA clone, 600,000 clones were generated and divided into subsets of approximately 16,000 clones per pool. Each of the 38 pools were grown overnight in L-broth in the presence of ampicillin and the plasmids were purified over a CsCl gradient.

### B. Screening for the P-selectin ligand protein gene

In the first stage,the LEC-γ1 binding assay of Example 4(A) was utilized to pan the HL60 cDNA library and thereby to enrich for the plasmid of interest. Six µg of each HL60 cDNA library pool was co-transfected with 2 µg of α 3/4FT gene (Example 2) into COS cells. Approximately 45 hours post-transfection, the COS cells were lifted from the plates by incubating the cells in 1mM EGTA for 15 min. at 37°C, followed by scraping with cell lifters. The cells were washed twice in Hanks buffered saline solution containing 1mM calcium (HBSS). The cells were resuspended in 4 ml of HBSS. The resuspended transfected COS cells were screened using the LEC-γ1 binding assay described in Example 4(A).

The plasmids from adherent COS cells were recovered from a Hirts extract [B. Hirts, J. Mol. Biol., 26, 365-369 (1967)] and then electroporated into E. coli DH5α cells for amplification. The enriched population of plasmids was purified over a CsCl gradient and re-transfected along with the 3/4FT gene (Example 2) into COS cells. The transfection, screening, and plasmid amplification process was repeated for a total of three times before a pool that bound to the LEC-γ1-coated plates was visually detected. The positive plasmid pool was subsequently broken down into subsets. This involved electroporating the Hirts extract from the positive pool into *E. coli* DH5α cells and quantitating colonies per ml as described above. Various pool sizes were produced by plating out a predetermined number of colonies on agar plates in the presence of ampicillin. Duplicate plates were prepared by performing nitrocellulose lifts and storing the filters on new agar plates. The duplicate plates served as reference plates for selecting individual or groups of colonies from any pool identified as being positive.

In the second stage of cloning, COS cells were co-transfected with the sublibrary pools and the 3/4FT gene by the same procedure used in the initial steps of screening. Forty-eight hours post-transfection, the transfected cells were screened using the fluorescent CHO:P-selectin assay of Example 4(B). Positive pools were further subdivided, as described above, until finally individual colonies were screened and positive clones identified. Using this method, a single positive clone, pMT21:PL85, was found to encode the P-selectin ligand protein. The DNA sequence of the P-selectin ligand contained in pMT21:PL85 is set forth in SEQ ID NO:1, and the binding characteristics of the P-selectin ligand protein encoded by pMT21:PL85 are set forth in Example 4(C) below.

### EXAMPLE 2

### CLONING THE α 1,3/1,4 FUCOSYLTRANSFERASE GENE

The α 1,3/1,4 fucosyltransferase gene (3/4FT) was cloned from total human genomic DNA (Clontech Laboratories) by means of PCR. The sense oligonucleotide primer contained an XbaI site and the 5' terminus of the gene (5'-TAGCATACGCTCTAGAGCATGGATCCCCTGGGTGCA GCCAAGC-3'), and the antisense oligonucleotide primer contained an EcoRI site and the 3' terminus of the gene (5'-CCGGAATTCTCAGGTGAA CCAAGCCGC-3'). The PCR product was sequentially digested with XbaI and EcoRI and purified by standard gel purification methods. This gene was then ligated with vector pMT3Sv2ADA (R. Kaufman, Methods in Enzymology, *supra*) that had also been sequentially digested with XbaI and EcoRI and purified by standard gel purification methods. Competent HB101 cells (Biorad) were transformed with this ligation product and then plated on agar plates in the presence of ampicillin. Nitrocellulose filter lifts of ampicillin-resistant transformants were probed with a radiolabeled oligonucleotide (5'-AAGTATCTGTCCAGGGCTTCCAGGT-3') complementary to the nucleotide region 506-530 in the middle of the gene (J. Sambrook et al., supra).

Plasmid DNA minipreps were prepared from twelve positive clones. The purified DNA was then digested with EcoRI and XbaI to identify the correct clone with the proper size insert. This clone (pEA. 3/4FT) was then grown up large scale and the DNA isolated by CsCl density gradient banding (J. Sambrook et al., *supra).* DNA sequencing confirmed the identity of the 3/4FT gene. The functionality of the gene was assessed in a cell-cell binding assay as follows. COS-1 monkey cells [(clone M6; M. Horwitz et al., Mol. Appl. Genet., 2:147-149, (1983)] were transfected with 3/4FT using DEAE dextran followed by DMSO shock treatment and chloroquine incubation [L. Sompeyrac and K. Dana, Proc. Natl. Acad. Sci., 78:7575-7578 (1981); M. Lopata et al., Nucleic Acids Res., 12:5707-5717, (1984); H. Luthman and G. Magnuson, Nucleic Acids Res., 11:1295-1308, (1983)]. The transfected COS cells were suspended and quantitated for binding to a CHO line expressing E-selectin [G. Larsen et al., J. Biol. Chem. 267:11104-11110, (1992)]. This assay confirmed that the COS cells transfected with 3/4FT can express the siaylated Lewis^{x} epitope on the cell surface.

### EXAMPLE 3

### EXPRESSION OF THE P-SELECTIN LIGAND PROTEIN

### A. Expression of the P-selectin Ligand in LEC11 cells

Functional P-selectin ligand was expressed in the SLe^{x}-positive Chinese hamster ovary (CHO) cell line LEC11 (Campbell, C. and Stanley, P.Cell 35:303-309 (1983) as follows: approximately 8 µg of plasmid containing the P-selectin ligand gene (pMT21:PL85, Example 1) was transfected into LEC11 cells. At 68 hours post-transfection, the cells were treated with 2.5 mM sodium butyrate for 4 hours. The cells were observed to induce P-selectin adhesion, as determined using the 6-CFD labeled CHO:P-selectin cell binding assay (described in Example 4, section B). In contrast, neither LEC11 cells alone nor LEC11 cells transfected with a control plasmid induced P-selectin adhesion.

### B. Expression of Soluble P-selectin Ligand in COS cells

COS cells were transfected with 8 µg pED.sPSL.T7 (see Example 5C) and 4 µg pEA.3/4 FT plasmid of Example 2, 8 µg pED.sPSL.T7 alone, or 8 µg plasmid vector (pMT21) and 4 µg pEA.3/4 FT gene. Forty-five hr post-transfection, the cells were rinsed twice in PBS and incubated overnight at 37°C in serum-free DMEM minus phenol red (JRH Biosciences) supplemented with 2 mM L-glutamine, 100 U/ml penicillin and 100 µg/ml streptomycin. Phenylmethylsulfonyl fluoride, aprotinin and NaN₃ were added to final concentrations of 1mM, 2 µg/ml and 0.02%, respectively, and the conditioned medium was centrifuged to remove all debris.

For immunoprecipitation experiments, the labeled soluble P-selectin ligand protein was produced by co-transfecting COS cells with pED.sPSL.T7 and pEA.3/4 FT. At forty-five hr post-transfection, the COS cells were labeled with 250 µCi/ml ³⁵S methionine (NEN) for 5 hours and the medium was collected. Expression of sPSL.T7 protein was confirmed by immunoprecipitation with anti-T7 antibodies.

### C. Expression of PACE-cleaved P-selectin ligand in COS Cells

COS cells were co-transfected with the pED.sPSL.T7 plasmid of Example 5(C), the pEA.3/4FT cDNA of Example 2, and a plasmid containing the PACE cDNA as set forth in SEQ ID NO:5. A parallel control co-transfection was done using only the pED.sPSL.T7 plasmid and the pEA.3/4FT plasmid. After 45 hours, conditioned medium from these transfected COS cells was coated onto plastic dishes and binding to CHO:P-selectin cells (Example 4) was determined. An approximately two-fold increase in bound CHO:P-selectin cells was observed for dishes coated with medium containing the P-selectin ligand co-expressed with PACE, as compared with medium containing P-selectin ligand which had not been co-expressed with PACE. Amino acid sequencing of the N-terminus of purified sPSL.T7 protein from the PACE co-transfection showed that all of the ligand had been cleaved at the PACE consensus site (amino acids 38-41 of SEQ ID NO:1). Radiolabeling of co-transfected COS cells with ³⁵S-methionine and subsequent SDS-polyacrylamide gel electrophoresis and autoradiography showed that comparable quantities of the P-selectin ligand had been secreted in both co-transfections.

### D. Expression of the P-selectin Ligand Protein in CHO cells

A full-length form (amino acids 1-402) of the P-selectin ligand protein was expressed in the CHO(DUKX) cell line (Urlaub & Chasin, Proc. Natl. Acad. sci.USA 77, 4216-4220 (1980)) as follows: approximately 25 µg of the pMT21:PL85 plasmid and approximately 8 µg of the pED.3/4FT (produced by restriction of pEA.3/4FT with EcoRI and XbaI and insertion of the resulting fragment into the pED plasmid) were co-transfected into CHO(DUKX) cells using the calcium phosphate method. Transfectants were selected for resistance to methotrexate. After two weeks, individual colonies were screened for SLe^{x} expression by using a conjugate of an anti SLe^{x} antibody (CSLEX-1, U.S. 4,752,569) and sheep red blood cells (sRBC) prepared by the chromic chloride method (Goding, J. W., J. Immunol. Methods 10:61-66 (1976) as follows: sRBC were washed with 0.15M NaCl until the wash became clear and then a 50% suspension of sRBC was prepared in 0.15M Nacl. One ml of 0.01% chromic chloride solution was added dropwise while vortexing to 0.2 ml of a sRBC suspension containing 50 µg of CSLEX-1. After incubating at 37°C for 30 minutes, 10 ml of phosphate buffered saline (PBS) solution was added to the reaction. The conjugate was washed once before resuspending into 10 ml of PBS. The plates containing transfectants were washed with PBS and then 3 ml of PBS and one ml of the sRBC/CSLEX-1 conjugate was added to each plate. Positive colonies were red on a transilluminator and were picked into alpha medium with 10% fetal bovine serum. After two weeks, colonies were subjected to stepwise amplification using methotrexate at concentrations of 2, 10, 25, 100, 250 nM. The stable cell line obtained was designated CD-PSGL-1 (R3.4). Expression of the P-selectin ligand protein was confirmed by immunoprecipitation studies using the polyclonal anti-P-selectin ligand protein antibody of Example 7(A). The functionality of the P-selectin ligand protein produced by the CD-PSGL-1 (R3.4) cell line was tested by assaying the transfectants for binding to LEC-γ1 as in Example 4(A).

The sPSL.T7 protein was expressed in a stable CHO-PACE line which was already expressing the cDNA encoding PACE as set forth in SEQ ID NO:5 under adenosine deaminase selection (Kaufman, et al., PNAS (USA) 83:3136-3140 (1986)). The psPSL.T7 (25 µg) and pED.3/4FT (8 µg) plasmids were cotransfected into CHO-PACE cells using the calcium phosphate method. Transfectants were selected for resistance to methotrexate, and individual colonies which bound to the sRBC/CSLEX-1 conjugate were picked. After two weeks in culture, the colonies were subjected to stepwise amplification as described above. The stable cell line obtained was designated CP/PSL-T7 (R4.1). Expression of sPSL.T7 protein was confirmed by standard immunoprecipitation methods using either a T7 specific monoclonal antibody or the LEC-γ1 chimera of Example 4(A). In a similar fashion, a stable cell line expressing the mature full length form (amino acids 42-402) of the P-selectin ligand protein was obtained by co-transfection of pMT21:PL85and pED.3/4FT into the CHO-PACE line.

Stable cell lines expressing the sPSL.Q protein of Example 5(B) and the sPSL.Fc protein of Example 5(D) were constructed as follows: plasmids pED.sPSL.Q (25 µg) or pED.sPSL.Fc (25 µg) were cotransfected with approximately 25 µg of the pED.3/4FT plasmid described above and approximately 20 µg of a plasmid containing the PACE cDNA as set forth in SEQ ID NO:5) as well as the neomycin resistance gene into CHO(DUKX) cells using the calcium phosphate method. Transfectants were selected for resistance to methotrexate and the G418 antibiotic. Approximately two weeks later, individual colonies were screened for SLe^{x} expression using sRBC/CSLEX-1 conjugate binding. The positive colonies were picked in G418 medium at 1 mg/ml concentration. After 2-3 weeks in culture, cells were amplified with methotrexate in a stepwise selection. The stable cell lines obtained were designated CD-sPSL.Q (R8.2) and CD-sPSL.Fc (R8.1), respectively. The expression of sPSL.Q and sPSL.Fc protein was confirmed by standard immunoprecipitation method using the anti P-selectin ligand protein polyclonal antibody of Example 7(A).

### EXAMPLE 4

### ASSAYS OF P-SELECTIN-MEDIATED INTERCELLULAR ADHESION

### A. LEC-γ1 Binding Assay

A DNA encoding a chimeric form of P-selectin conjugated to the Fc portion of a human IgGγ1 (LEC-γ1) was constructed using known methods (Aruffo et al. Cell 67, 35-44 (1991)), and stably transfected into dhfr⁻ CHO cells (CHO DUKX) for high level production of the chimeric LEC-γ1 protein, which was purified for use in the binding assay set forth below.

Petri dishes were coated first with a polyclonal anti-human IgGγ1 Fc antibody and then with LEC-γ1. This method orients the LEC-γ1 construct such that the P-selectin portion of the chimeric molecule is presented on the surface of the plates. Adhesion of HL60 cells to the oriented LEC-γ1 was quantitated in the presence and absence of calcium. HL60 adhesion was shown to be calcium dependent, confirming that the chimeric molecule had retained functional binding of P-selectin to its ligand on HL60 cells. The binding of HL60 cells to oriented LEC-γ1 was also shown to be blocked by a neutralizing monoclonal antibody to P-selectin, demonstrating the specificity of P-selectin binding.

### B. Fluorescent CHO-P-selectin Binding Assay

The assay employed a fluorescently labeled CHO:P-selectin cell line (Larsen et al., J. Biol. Chem. 267, 11104-11110 (1992)) that can bind to and form clusters on the surface of COS cells that are co-transfected with the P-selectin ligand gene and the 3/4 FT gene. The CHO:P-selectin cells were suspended at 1.5 x 10⁶ cells/ml in 1% fetal bovine serum in DME medium and labeled by adding 6-carboxyfluorescein diacetate (6-CFD) to a final concentration of 100 ug/ml. After incubation at 37°C for 15 minutes, the cells were washed in medium and resuspended at 1 x 10⁵ cells/ml. Five ml of the labeled cells were added to each washed COS transfectant-containing plate to be assayed and incubated at room temperature for 10 minutes. Nonadherent cells were removed by four washes with medium. The plates were then scanned by fluorescence microscopy for rosettes of adherent CHO:P-selectin cells.

### C. Quantitative adhesion assay using radioactively labeled CHO:P-selectin cells

COS cells were co-transfected with the pMT21:PL85 plasmid of Example 1 and the pEA.3/4FT plasmid of Example 2 by the same procedure used in the initial stages of screening. As controls, COS cells were transfected with pMT21:PL85 alone, or with pEA.3/4FT alone, or with a similar plasmid containing no insert ("mock"). 24 hours post-transfection, the transfected cells were trypsinized and distributed into Costar 6-well tissue culture plates. CHO:P-selectin cells were labeled for 16 hours with ³H-thymidine using known methods and preincubated at 0.5 x 10⁶ cells/ml for 30 minutes at 4°C in a medium containing 1% BSA (control); a medium containing 1% BSA, 5 mM EDTA and 5 mM EGTA; α medium containing 1% BSA and 10 µg/ml of a neutralizing anti P-selectin monoclonal antibody; and a medium containing 1% BSA and a non-neutralizing anti-P-selectin monoclonal antibody. The preincubated cells were then added to the wells containing the transfected COS cells. After a 10 minute incubation, unbound cells were removed by 4 changes of medium. The bound CHO:P-selectin cells were released by trypsinization and quantified by scintillation counting.

COS cells co-transfected with P-selectin ligand and the 3/4 FT induced approximately 5.4-fold more binding of CHO:P-selectin cells relative to COS mock cells; assay in the presence of EGTA and EDTA reduced binding to the level of the mock transfected COS cells. Likewise, incubation with neutralizing anti-P-selectin antibody also eliminated specific binding, whereas non-neutralizing antibody had no effect. In contrast, the binding of CHO:P-selectin to COS cells transfected with P-selectin ligand alone was not statistically different than binding to the mock-transfected COS in both the presence or absence of EDTA and EGTA, or anti-P-selectin antibodies. The binding of CHO:P-selectin cells to COS cells transfected with 3/4 FT alone was approximately 2-fold greater than to the mock-transfected COS, but was unaffected by the presence or absence of EDTA and EGTA.

### EXAMPLE 5

### CONSTRUCTION OP SOLUBLE P-SELECTIN LIGANDS

The EcoRI adaptors used to generate the cDNA library from HL60 cells in Example I contain an XbaI restriction site (TCTAGA) just 5' of the beginning of SEQ ID NO:1 as it is located in the pMT21:PL85 plasmid. In order to generate soluble forms of the PSL, the pMT21:PL85 plasmid was restricted with XbaI and with HincII (which cleaves after nucleotide 944 of SEQ ID NO:1). The approximately 950 bp fragment thus generated, containing all of the encoded extracellular segment of the ligand up to and including the codon for valine 295, was isolated and used to generate DNAs encoding soluble forms of the P-selectin ligand protein as set forth in sections A though D below.

### A. Construction of psPSL.OC

The fragment was purified and ligated into mammalian expression vector pED between the XbaI and EcoRI sites, along with double stranded synthetic oligonucleotide DNA that recreated the codons from Asn 296 to Cys 310 and introduced a novel stop codon immediately following Cys 310. The sequence of the oligos is as follows:
5'-AACTACCCAGTGGGAGCACCAGACCACATCTCTGTGAAGCAGTGCTAG
5'-AATTCTAGCACTGCTTCACAGAGATGTGGTCTGGTGCTCCCACTGGGTAGTT
The resulting plasmid was designated pED.sPSL.QC, and the protein expressed from the plasmid was designated sPSL.QC.

### B. construction of psPSL.O

The fragment was purified and ligated into the pED plasmid (Kaufman et al., 1991) between the XbaI and EcoRI sites, along with the double stranded synthetic oligonucleotide DNA that recreated the codons from Asn 296 to Gln 309 and introduced a novel stop codon immediately following Gln 309. The sequence of the oligos is as follows:
5'-AACTACCCAGTGGGAGCACCAGACCACATCTGGTGAAGCAGTAG
5'-AATTCTACTGCTTCACAGAGATGTGGTCTGGTGCTCCCACTGGGTAGTT
The resulting plasmid was designated pED.sPSL.Q, and the protein expressed from the plasmid was designated sPSL.Q.

### C. Construction of psPSL.T7

Oligonucleotides encoding 14 amino acids including an epitope derived from the phage T7 major capsid protein were synthesized, creating a C-terminal fusion of the epitope "tag" with an additional 32 amino acids derived from the vector sequence. Two oligonucleotides having the sequences
5'-CTAGACCCGGGATGGCATCCATGACAGGAGGACAACAAATGGTAGGCCGTAG and
5'-AATTCTACGGCCTACCCATTTGTTGTCCTCCTGTCATGGATGCCATCCCGGGT
were duplexed and ligated with the large XbaI-EcoRI fragment of mammalian expression plasmid pED. The resulting plasmid, pED.T7 was restricted with XbaI and SmaI and ligated to the 950 bp XbaI-HincII fragment described above, resulting in plasmid pED.sPSL.T7.
The protein resulting from expression of pED.sPSL.T7 was designated sPSL.T7.

### D. construction of soluble P-selectin_Ligand--IgGFα Chimera

The plasmid DNA encoding a soluble, extracellular form of the P-selectin ligand protein fused to the Fc portion of human immunoglobulin IgG1 was constructed as follows: the mammalian expression vector pED.Fc contains sequences encoding the Fc region of a human IgG1 with a novel linker sequence enabling the fusion of coding sequences amino terminal to the hinge region via a unique XbaI restriction site. A three fragment ligation was performed: pED.Fc was restricted with XbaI and gel purified in linear form. The 950 bp fragment from pMT21:PL85 described above comprised the second fragment. The third fragment consisted of annealed synthetic oligonucleotide DNAs having the following sequence:
5' - CTGCGGCCGCAGT
5' - CTAGACTGCGGCCGCAG
The ligation products were grown as plasmid DNAs and individual clones having the correct configuration were identified by DNA sequencing. The plasmid was designated pED.PSL.Fc. The DNA coding region of the resulting soluble P-selectin ligand /Fc fusion protein is shown in SEQ ID NO:6

### EXAMPLE 6

### CHARACTERIZATION OF EXPRESSED P-SELECTIN LIGANDS

### A. Binding Characterization of Full-Length P-selectin Ligand Protein Expressed on COS Cells

Co-transfection of COS cells with the pEA.3/4FT plasmid of Example 2 and the pMT21:PL85 plasmid of Example 1 yields COS cells which specifically bind to CHO:P-selectin cells. This binding is observed only upon co-transfection of pEA.3/4FT and pMT21:PL85; use of either plasmid alone generates COS cells which do not bind to CHO:P-selectin cells. No binding is observed between the parental CHO(DUKX) cell line which does not express P-selectin and COS cells co-transfected with pEA.3/4FT and pMT21:PL85. The binding between the co-transfected COS cells and CHO:P-selectin cells is sensitive to chelators of divalent ions such as EDTA and EGTA, consistent with the Ca⁺⁺ dependency of P-selectin mediated cellular adhesion. A neutralizing anti-P-selectin monoclonal antibody blocked the binding between the CHO:P-selectin cells and the COS cells which had been cotransfected with pEA.3/4FT and pMT21:PL85, while a non-neutralizing anti-P-selectin monoclonal antibody had no effect on the binding. The antibody results indicate that the functional domain(s) of P-selectin are required for binding to P-selectin ligand protein expressed on the surface of COS cells.

### B. Electrophoretic Characterization of Full-Length P-selectin Ligand Expressed in COS Cells

Detergent extracts of co-transfected COS cells were prepared as follows: 45 hours post co-transfection, approximately 1.5 x 10⁷ cells were suspended in 5 ml of lysis buffer (10mM Piperazine-N,N-bis[2-ethanesulfonic acid] (PIPES) pH 7.5, 100 mM KCl, 3 mM MgCl₂, 1 mM benzamidine, 0.5 µg/ml leupeptin, 0.75 µg/ml pepstatin, 1 mM ethylmaleimide, and 1 µg/ml aprotinin) and lysed by sonication. Cellular debris was removed by low speed centrifigation (500 x g. 10 minutes), and a membrane fraction collected by ultracentrifugation (100,000 x g, 60 min). The high speed membrane pellet was resuspended in an extraction buffer (10 mM 3-[N-Morpholino]propanesulfonic acid) (MOPS) pH 7.5, 0.1M NaCl, 0.02% NaN₃, 1% Thesit® (Sigma), 1 mM benzamidine, 0.5 µg/ml leupeptin, 0.75 µg/ml pepstatin, 1 mM ethylmaleimide, and 1 µg/ml aprotinin). Samples were then subjected to SDS polyacrylamide gel electrophoresis and transfer to nitrocellulose blots as follows: an aliquot of the detergent extract was suspended in 1% SDS loading buffer and heated for 5 minutes at 100°C before loading onto an 8-16% polyacrylamide gel (reduced) or a 6% gel (non-reduced) and electrophoresed in the Laemmli buffer system. Blots were prepared using Immobilon-P® transfer membranes. The blots were immersed in 10 mM MOPS pH 7.5, 0.1M NaCl, 0.02% NaN₃, 1 mM MgCl₂, 1 mM CaCl₂, and 10% non-fat milk overnight at 4°C. Blots were rinsed once in the above buffer, minus the milk, and incubated in blotting buffer (10 mMMOPS pH 7.5, 0.1M NaCl, 1% bovine serum albumin, 0.05% Thesit, 1 mM MgCl₂, 1 mM CaCl₂) for 30 minutes at room temperature.

The blots were then probed for the P-selectin ligand as follows: 50 ng of a P-selectin/Fc chimera was pre-incubated with 3 µCi of ¹²⁵I-Protein A in blotting buffer for 30 minutes at room temperature. Additional excipients (e.g., EDTA, EGTA, monoclonal antibodies) could be added to the pre-incubation mixture at this point to evaluate their effects on binding of the chimera to the P-selectin ligand. The pre-incubated mixture was then incubated with the blots (prepared as above) for 60 minutes at room temperature, and the blots were subsequently washed four times with the same blotting buffer (without bovine serum albumin), air dried, and autoradiographed at -70°C.

Under non-reducing conditions, two bands were observed with this technique for membrane extracts prepared from co-transfected COS cells. The major band migrated with an estimated molecular weight of approximately 220 kD, whereas the minor band migrated with a molecular weight of approximately 110 kD. Under reducing conditions, only a single band was observed with a molecular weight of approximately 110 kD, indicating that under non-reducing conditions, the P-selectin ligand exists as a homodimer. The approximate molecular weight of the reduced monomer is greater than that predicted from the deduced amino acid sequence of the cDNA clone (45 kD), indicating that the expressed protein undergoes extensive post-translational modification (see Example 6(C)). The specificity of the P-selectin/Fc chimera was confirmed by the observation that a nonspecific IgG₁ probe yielded no bands on the blots. Additionally, the binding of the P-selectin/Fc chimera to the blots was abolished by EDTA, EGTA, and a neutralizing anti-P-selectin monoclonal antibody. Specific bands on the blots were observed only from membrane extracts of COS cells co-transfected with the pEA.3/4FT and pMT21:PL85 plasmids. Membrane extracts from control transfections (pEA.3/4FT or pMT21:PL85 alone) failed to yield observable bands on blots.

### C. Glycosylation of p-selectin Ligand Protein

The presence of covalently attached carbohydrate on recombinant P-selectin ligand and its role in binding to P-selectin was determined as follows: COS cells were cotransfected with pED.sPSL.T7 of Example 5(C) and the pEA.3/4FT plasmid of Example 2. After 48 hours, the cells were pulsed with ³⁵S-methionine. 200 µl of ³⁵S methionine-labeled sPSL.T7 conditioned medium was incubated with 5 µg LEC-γ1 in the presence of 2mM CaCl₂ and 1 mg/ml bovine serum albumin (BSA). After rotating for 2 hours at 4°C, Protein A-Sepharose beads (Pharmacia) were added for 1 hour at 4°C, pelleted by centrifugation and washed twice in Tris buffered saline (20 mM Tris-HCl, 150 mM NaCl pH 7.5, hereinafter TBS) containing 2mM CaCl₂ and 1 mg/ml BSA. The pellets were then resuspended and treated with neuraminidase (*Streptococcus pneumoniae*)*,* O-glycanase, and N-glycanase (all from Genzyme) as follows. All glycosidase digestions were done at 37°C overnight. For neuraminidase digestion, the pellet was resuspended in 50 µl 2-(N-morpholino)-ethanesulfonic acid (MES) buffer, pH 6.5 (Calbiochem) and 0.1% SDS, heated at 95°C for 5 minutes, then pelleted. The supernatant was modified to contain 1.4% n-Octyl B-D-glucopyranoside (OGP), 10mM calcium acetate, 20 mM sodium cacodylate and 2.5 mM PMSF, final pH 7.0 Eight µl neuraminidase was added for a final concentration of 1 unit/ml. For neuraminidase/O-glycanase digestion, the sample was prepared as above and along with the neuraminidase, the O-glycanase was also added to a final concentration of 0.1 unit/ml. For N-glycanase digestion, the pellet was resuspended in 54 ul MES buffer and 1% SDS, heated at 95°C for 5 minutes, then pelleted. The supernatant was modified to contain 0.2 M sodium phosphate, 3.5% OGP, and 2.5 mM PMSF, final pH 8.5. N-glycanase was added for a final concentration of 12 units/ml and incubated as above.

The effect of glycosidase treatment on sPSL.T7 was assessed in two ways. For this, each digested protein sample was divided into two equal fractions. One fraction was precipitated with the P-selectin polyclonal antibody of Example 7(A), to show the effect of digestion on the electrophoretic mobility. The other fraction was precipitated with the LEC-γ1 chimera of Example 4(A), to assess the remaining P-selectin ligand binding activity after digestion. The immunoprecipitationed samples were analyzed by SDS-polyacrylamide gel electrophoresis under reducing conditions and autoradiography.

In the absence of glycosidase treatment, autoradiography revealed comparable bands (with molecular weights of 110 kD) for each precipitation. When the P-selectin ligand protein was treated with neuraminidase, anti-P-selectin ligand polyclonal antibody precipitation revealed a slight decrease in mobility, consistent with removal of sialic acid residues. The amount of P-selectin ligand protein precipitated by LEC-γ1 was significantly reduced after neuraminidase treatment, consistent with the role of sialic acid residues in the P-selectin/P-selectin ligand interaction. When the P-selectin ligand protein was treated with both neuraminidase and O-glycanase, a substantial increase in electrophoretic mobility was observed after precipitation with the anti-P-selectin ligand polyclonal antibody, indicating that a number of O-linked oligosaccharide chains had been removed. However, removal of O-linked oligosaccharides from the P-selectin ligand protein may not have been complete, since the electrophoretic mobility did not correspond to a protein with a molecular weight of 38 kD, as would be predicted from the amino acid sequence set forth in SEQ ID NO:1. The neuraminidass/O-glycanase digested P-selectin ligand protein bound to LEC-γ1 very poorly, further indicating the role of oligosaccharides in the P-selectin/P-selectin ligand interaction. Treatment of the purified P-selectin ligand with N-glycanase resulted in a slight increase in electrophoretic mobility, demonstrating that some of the consensus sites for N-linked glycosylation are occupied. The amount of P-selectin ligand protein precipitated by LEC-γ1 was slightly reduced, indicating that N-linked glycosylation also contributes to the P-selectin/P-selectin ligand interaction, though not as dramatically as sialylation and o-linked glycosylation.

### EXAMPLE 7

### POLYCLONAL ANTIBODIES SPECIFIC FOR P-SELECTIN LIGANDS

### A. Polyclonal Rabbit anti-P-selectin Ligand Protein/Maltose Binding Protein Fusion Protein

The anti-P-selectin ligand polyclonal antibody was generated by immunizing rabbits with a fusion protein generated in *E. coli*. The fusion protein consisted of the amino terminal one-third of the P-selectin ligand (amino acids 1 to 110 of SEQ ID NO:1) fused in frame to the maltose binding protein (Maina, C. V. et al., Gene 74, 365-373 (1988); Riggs, P., in Current Protocols in Molecular Biology, F. M. Ausebel et al., Eds., Greene Associates/Wiley Interscience (New York, 1990) chapter 16.6). Under conditions employed herein, the fusion protein antibody recognizes the P-selectin ligand protein.

### B. Polyclonal Rabbit Anti-sPSL.T7 Protein

A soluble form of the invention (sPSL.T7; see example 5(C)) was purified to apparent homogeneity according to the following scheme: COS cells were transfected with three plasmids, one encoding each of the following: sPSL.T7 (Example 5(C)), 3/4FT (Example 2), and a soluble form of PACE (as set forth in SEQ ID NO:5). After 72 hours, the conditioned medium was collected and recombinant sPSL.T7 was purified as follows.

Conditioned medium was diluted two fold with 50 mM MOPS, 150 mM NaCl, 0.5 mM CaCl₂and 0.5 mM MnCl₂, pH 7.2, and applied to a column of lentil lectin-Sepharose 4B equilibrated in the same buffer. After loading, the column was washed with the same buffer until the optical absorbance at 280 nm dropped to a stable baseline. The column was then eluted with the same buffer which had been adjusted to 0.5 M α-methyl-mannoside and 0.3 M NaCl. Recombinant sPSL.T7 was collected over 5-15 column volumes of this elution buffer. The lentil lectin eluate was then subjected to a 0-70% ammonium sulfate precipitation by adding 472g of ammonium sulfate per liter of column eluate at 4°C. After stirring for 30 minutes, the precipitate was resuspended in a minimal volume of TBS (20 mM Tris-HCl, 150 mM NaCl, pH 7.5) and applied to a TSK G4000SW_{XL} gel filtration column equilibrated in TBS. The flow rate on the column was 0.5 ml/min and a guard column was employed. In aliquots of < 250 µl, the resuspended ammonium sulfate pellet was injected on the column and fractions analyzed by SDS-PAGE with Western analysis. Fractions containing sPLS.T7 were pooled and then used for immunizing rabbits.

Antibodies to sPSL.T7 were generated in the standard fashion by antigen priming and subsequent boosting over a 3 month period. Specifically, primary immunization was performed by mixing 50 µg of sPSL.T7 (denatured by mixing in 0.1% SDS and heating for 10 minutes at 100°C) with complete Freund's adjuvant and injected at five sites subcutaneously. The second (and all subsequent) boosts were performed by mixing 25 µg of sPSL.T7 (denatured by mixing in 0.1% SDS and heating for 10 minutes at 100°C) [12.5 µg for the third and subsequent boosts] with incomplete Freund's adjuvant and injecting at two sites subcutaneously (or later, intramuscularly) every two weeks. Test bleeds were performed every two weeks to monitor antibody titer. When the antibody titer reached a suitable level, a larger scale bleed was performed and a total serum fraction prepared. This polyclonal antibody preparation was used to inhibit the specific binding of HL60 cells to CHO:P-selectin cells in a manner similar to that described in Example 4.

This assay employed fluorescently-labeled HL60 cells (labelled with BCECFAM; 2',7'-bis-(2-carboxymethyl)-5-(and-6)-carboxyfluorescein, acetoxymethyl ester) binding to CHO cells plated on the bottom of microtiter plates. The labelled HL60 cells were pre-incubated with either sera containing polyclonal antibody or with pre-immune sera for 30 minutes at 4°C. The cells were then washed and incubated with the CHO:P-selectin cells for 10 minutes. The plates were then washed and the fluorescence read with a fluorescence microtiter plate reader. Using this assay, a 1:15 dilution of the anti-sPSL.T7 polyclonal serum resulted in essentially complete inhibition of HL60 cell binding to CHO:P-selectin. Demonstrable inhibition of HL60 binding to CHO:P-selectin was still observed at antiserum dilutions of 1:150. Pre-immune serum had no effect on HL60 cell binding to CHO:P-selectin

## Claims

1. A fragment of P-selectin ligand protein having P-selectin ligand activity comprising an amino acid sequence selected from:
a) amino acid 21 to amino acid 54 of SEQ ID NO:1
b) amino acid 55 to amino acid 127 of SEQ ID NO:1
c) amino acid 128 to amino acid 267 of SEQ ID NO:1
d) amino acid 268 to amino acid 308 of SEQ ID NO:1
e) amino acid 309 to amino acid 333 of SEQ ID NO:1
f) amino acid 334 to amino acid 402 of SEQ ID NO:1
g) amino acid 43 to amino acid 56 of SEQ ID NO:1
h) amino acid 1 to amino acid 310 of SEQ ID NO:1.

2. The fragment of P-selectin ligand protein comprising amino acid 43 to amino acid 56 of SEQ ID NO:1, wherein one or more of the tyrosines at amino acid 46, amino acid 48 and/or amino acid 51 is sulfated.

3. The P-selectin ligand of claim 1, wherein the P-selectin ligand is in a linear form.

4. The P-selectin ligand fragment of claim 1, wherein the P-selectin ligand is in a cyclic form.

5. A nucleic acid molecule comprising a sequence encoding one of the P-selectin ligand fragments of claim 1.

6. An antibody that specifically reacts with P-selectin ligand, wherein the sequence of the P-selectin ligand protein consists essentially of an amino acid sequence selected from:
a) amino acid 42 to amino acid 56 of SEQ ID NO: 1; and
b) amino acid 127 to amino acid 138 of SEQ ID NO: 1.

7. A plasmid encoding a P-selectin ligand comprising a sequence according to SEQ ID NO:1.

8. The plasmid of claim 7, wherein said plasmid is pMT21:PL85.

9. An antibody of claim 6 that specifically reacts with P-selectin ligand protein for use for treating a condition **characterized by** P-selectin-mediated adhesion.

10. Use of an antibody of claim 6 that specifically reacts with P-selectin-ligand protein for the preparation of a pharmaceutical composition for treating a condition **characterized by** P-selectin-mediated adhesion.

11. An antibody of claim 6 that specifically reacts with P-selectin ligand protein for use for the treatment or diagnosis of inflammatory diseases or cancer.

12. Use of an antibody of claim 6 that specifically reacts with P-selectin ligand protein for the preparation of a pharmaceutical composition for treating or diagnosing inflammatory diseases or cancer.

13. The antibody of claims 9 or 11 for the use specified therein, or the use of claims 10 or 12, wherein said antibody is a monoclonal antibody

14. A fusion protein comprising:
a) a first amino acid sequence chosen from: amino acid 21 to amino acid 54 of SEQ ID NO:1; amino acid 55 to amino acid 127 of SEQ ID NO:1; amino acid 128 to amino acid 267 of SEQ ID NO:1; amino acid 268 to amino acid 308 of SEQ ID NO:1; amino acid 309 to amino acid 333 of SEQ ID NO:1; amino acid 334 to amino acid 402 of SEQ ID NO:1; amino acid 43 to amino acid 56 of SEQ ID NO:1; and amino acid 42 to amino acid 295 of SEQ ID NO:1; and
b) a second amino acid sequence derived from the sequence of an Fc portion of an immunoglobulin.

15. The fusion protein of claim14, wherein the first amino acid sequence comprises amino acid 43 to amino acid 56 of SEQ ID NO:1,

16. The fusion protein of claim 14 or claim 15, wherein the immunoglobulin is an IgG molecule.

17. The fusion protein of claim 16, wherein the IgG is IgG₁.

18. The fusion protein of claim 14 or claim 15, wherein the immunoglobulin is an IgM molecule.

19. The fusion protein of claim 15, wherein one or more tyrosines at amino acid 46, amino acid 48 and/or amino acid 51 of the first amino acid sequence are sulfated.

20. A DNA encoding the fusion protein of claim 14 or claim 15.

21. A plasmid comprising a DNA encoding a soluble P-selectin ligand-Fc fusion protein.

22. The plasmid of claim 21, wherein the plasmid is pED.PSL.Fc.

## Patentansprüche

1. Fragment des P-Selectin-Ligandenproteins mit P-Selectin-Ligandenaktivität, umfassend eine Aminosäuresequenz, ausgewählt aus:
a) Aminosäure 21 bis Aminosäure 54 der SEQ ID NO:1;
b) Aminosäure 55 bis Aminosäure 127 der SEQ ID NO:1;
c) Aminosäure 128 bis Aminosäure 267 der SEQ ID NO:1;
d) Aminosäure 268 bis Aminosäure 308 der SEQ ID NO:1;
e) Aminosäure 309 bis Aminosäure 333 der SEQ ID NO:1;
f) Aminosäure 334 bis Aminosäure 402 der SEQ ID NO:1;
g) Aminosäure 43 bis Aminosäure 56 der SEQ ID NO:1;
h) Aminosäure 1 bis Aminosäure 310 der SEQ ID NO:1.

2. Fragment des P-Selectin-Ligandenproteins, umfassend Aminosäure 43 bis Aminosäure 56 der SEQ ID NO:1, wobei eines oder mehrere der Tyrosine bei Aminosäure 46, Aminosäure 48 und/oder Aminosäure 51 sulfatiert sind.

3. P-Selectin-Ligand nach Anspruch 1, wobei der P-Selectin-Ligand in einer linearen Form vorliegt.

4. Fragment des P-Selectin-Liganden nach Anspruch 1, wobei der P-Selectin-Ligand in einer zyklischen Form vorliegt.

5. Nukleinsäuremolekül, umfassend eine Sequenz, kodierend für eines der Fragmente des P-Selectin-Liganden nach Anspruch 1.

6. Antikörper, der spezifisch mit einem P-Selectin-Liganden reagiert, wobei die Sequenz des P-Selectin-Ligandenproteins im Wesentlichen aus einer Aminosäuresequenz besteht, die ausgewählt ist aus:
a) Aminosäure 42 bis Aminosäure 56 der SEQ ID NO:1 und
b) Aminosäure 127 bis Aminosäure 138 der SEQ ID NO:1.

7. Plasmid, kodierend für einen P-Selectin-Liganden, umfassend eine Sequenz gemäß SEQ ID NO:1.

8. Plasmid nach Anspruch 7, wobei das Plasmid pMT21 :PL85 ist.

9. Antikörper nach Anspruch 6, der spezifisch mit einem P-Selectin-Ligandenprotein reagiert, zur Verwendung bei der Behandlung eines Zustands, der durch P-Selectin-vermittelte Adhäsion **gekennzeichnet** ist.

10. Verwendung eines Antikörpers nach Anspruch 6, der spezifisch mit einem P-Selectin-Ligandenprotein reagiert, zur Herstellung einer pharmazeutischen Zusammensetzung zur Behandlung eines Zustands, **gekennzeichnet durch** P-Selectin vermittelte Adhäsion.

11. Antikörper nach Anspruch 6, der spezifisch mit einem P-Selectin-Ligandenprotein reagiert, zur Behandlung oder Diagnose von entzündlichen Erkrankungen oder Krebs.

12. Verwendung eines Antikörpers nach Anspruch 6, der spezifisch mit einem P-Selectin-Ligandenprotein reagiert, zur Herstellung einer pharmazeutischen Zusammensetzung zur Behandlung oder Diagnose von entzündlichen Erkrankungen oder Krebs.

13. Antikörper nach Anspruch 9 oder 11 zur darin spezifizierten Verwendung oder zur Verwendung nach Anspruch 10 oder 12, wobei der Antikörper ein monoklonaler Antikörper ist.

14. Fusionsprotein, umfassend:
a) eine erste Aminosäuresequenz, ausgewählt aus: Aminosäure 21 bis Aminosäure 54 der SEQ ID NO:1; Aminosäure 55 bis Aminosäure 127 der SEQ ID NO:1; Aminosäure 128 bis Aminosäure 267 der SEQ ID NO:1; Aminosäure 268 bis Aminosäure 308 der SEQ ID NO:1; Aminosäure 309 bis Aminosäure 333 der SEQ ID NO:1; Aminosäure 334 bis Aminosäure 402 der SEQ ID NO:1; Aminosäure 43 bis Aminosäure 56 der SEQ ID NO:1; and Aminosäure 42 bis Aminosäure 295 der SEQ ID NO:1; und
b) eine zweite Aminosäuresequenz, abgleitet aus der Sequenz eines Fc-Abschnitts eines Immunglobulins.

15. Fusionsprotein nach Anspruch 14, wobei die erste Aminosäuresequenz Aminosäure 43 bis Aminosäure 56 der SEQ ID NO:1 umfasst.

16. Fusionsprotein nach Anspruch 14 oder Anspruch 15, wobei das Immunglobulin ein IgG-Molekül ist.

17. Fusionsprotein nach Anspruch 16, wobei das IgG IgG₁ ist.

18. Fusionsprotein nach Anspruch 14 oder Anspruch 15, wobei das Immunglobulin ein IgM-Molekül ist.

19. Fusionsprotein nach Anspruch 15, wobei eines oder mehrere der Tyrosine bei Aminosäure 46, Aminosäure 48 und/oder Aminosäure 51 der ersten Aminosäuresequenz sulfatiert sind.

20. DNA, kodierend für das Fusionsprotein nach Anspruch 14 oder Anspruch 15.

21. Plasmid, umfassend eine DNA, kodierend für ein lösliches P-Selectin-Liganden-Fc-Fusionsprotein.

22. Plasmid nach Anspruch 21, wobei das Plasmid pED.PSL.Fc ist.

## Revendications

1. Fragment de protéine de ligand P-selectin présentant une activité de ligand P-selectin comprenant une séquence d'acide aminés sélectionnés parmi :
a) acide aminé 21 à acide aminé 54 de SEQ ID NO:1;
b) acide aminé 55 à acide aminé 127 de SEQ ID NO:1;
c) acide aminé 128 à acide aminé 267 de SEQ ID NO:1;
d) acide aminé 268 à acide aminé 308 de SEQ ID NO:1;
e) acide aminé 309 à acide aminé 333 de SEQ ID NO:1;
f) acide aminé 334 à acide aminé 402 de SEQ ID NO:1;
g) acide aminé 43 à acide aminé 56 de SEQ ID NO:1;
h) acide aminé 1 à acide aminé 310 de SEQ ID NO:1.

2. Fragment de protéine de ligand P-selectin comprenant l'acide aminé 43 à l'acide aminé 56 de SEQ ID NO:1; dans lequel une ou plusieurs des tyrosines à l'acide aminé 46, l'acide aminé 48 et/ou l'acide aminé 51 est (sont) sulfatée(s).

3. Ligand P-selectin selon la revendication 1, dans lequel le ligand P-selectin est présent sous une forme linéaire.

4. Ligand P-selectin selon la revendication 1, dans lequel le ligand P-selectin est présent sous une forme cyclique.

5. Molécule d'acide nucléique comprenant une séquence codant pour un des fragments de ligand P-selectin selon la revendication 1.

6. Anticorps qui réagit spécifiquement avec le ligand P-selectin, dans lequel la séquence de protéine de ligand P-selectin consiste essentiellement en une séquence d'acides aminés sélectionnée parmi:
a) acide aminé 42 à acide aminé 56 de SEQ ID NO:1; et
b) acide aminé 127 à acide aminé 138 de SEQ ID NO:1.

7. Plasmide codant pour un ligand P-selectin, comprenant une séquence selon SEQ ID NO:1.

8. Plasmide selon la revendication 7, dans lequel ledit plasmide pMT21:PL85.

9. Anticorps selon la revendication 6 qui réagit spécifiquement avec la protéine de ligand P-selectin pour utilisation dans le traitement d'une condition **caractérisée par** adhésion étant dû au P-selectin.

10. Anticorps selon la revendication 6 qui réagit spécifiquement avec la protéine de ligand P-selectin pour utilisation dans le traitement d'une condition **caractérisée par** adhésion étant dû au P-selectin.

11. Anticorps selon la revendication 6 qui réagit spécifiquement avec la protéine de ligand P-selectin pour utilisation dans le traitement ou le diagnostic de maladies inflammatoires ou d'un cancer.

12. Anticorps selon la revendication 6 qui réagit spécifiquement avec la protéine de ligand P-selectin pour la préparation d'une composition pharmaceutique pour le traitement ou le diagnostic de maladies inflammatoires ou d'un cancer.

13. Anticorps selon les revendications 9 ou 11 pour l'utilisation qui y est spécifiée, ou l'utilisation selon les revendications 10 ou 12, dans laquelle ledit anticorps est un anticorps monoclonal.

14. Protéine de fusion comprenant :
a) une première séquence d'acides aminés choisis parmi : acide aminé 21 à acide aminé 54 de SEQ ID NO:1; acide aminé 55 à acide aminé 127 de SEQ ID NO:1; acide aminé 128 à acide aminé 267 de SEQ ID NO:1; acide aminé 268 à acide aminé 308 de SEQ ID NO:1; acide aminé 309 à acide aminé 333 de SEQ ID NO:1; acide aminé 334 à acide aminé 402 de SEQ ID NO:1; acide aminé 43 à acide aminé 56 de SEQ ID NO:1; et acide aminé 42 à acide aminé 295 de SEQ ID NO:1; et
b) une seconde séquence d'acides aminés dérivée de la séquence d'une partie Fc d'une immunoglobuline.

15. Protéine de fusion selon la revendication 14, dans laquelle la première séquence d'acides aminés comprend l'acide aminé 43 à l'acide aminé 56 de SEQ ID NO:1.

16. Protéine de fusion selon la revendication 14 ou la revendication 15, dans laquelle l'immunoglobuline est une molécule IgG.

17. Protéine de fusion selon la revendication 16, dans laquelle l'IgG est IgG₁.

18. Protéine de fusion selon la revendication 14 ou la revendication 15, dans laquelle l'immunoglobuline est une molécule IgM.

19. Protéine de fusion selon la revendication 15, dans laquelle une ou plusieurs tyrosines à l'acide aminé 46, l'acide aminé 48 et/ou l'acide aminé 51 de la première séquence d'acides aminés est (sont) sulfatée(s).

20. ADN codant pour la protéine de fusion selon la revendication 14 ou la revendication 15.

21. Plasmide comprenant un ADN codant pour une protéine de fusion Fc à ligand P-selectin soluble.

22. Plasmide selon la revendication 21, le plasmide étant pED.PSL.Fc.


### SEQUENCE LISTING

(1) GENERAL INFORMATION:
   (i) APPLICANT: GENETICS INSTITUTE, INC.
   (ii) TITLE OF INVENTION: NOVEL P-SELECTIN LIGAND PROTEIN
   (iii) NUMBER OF SEQUENCES: 6
   (iv) CORRESPONDENCE ADDRESS:
      (A) ADDRESSEE: Legal Affairs
      (B) STREET: 87 CambridgePark Drive
      (C) CITY: Cambridge
      (D) STATE: MA
      (E) COUNTRY: USA
      (F) ZIP: 02140
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: US 07/965,662 (B) FILING DATE: 23-OCT-1992 (C) APPLICATION NUMBER: US 08/112,608 (D) FILING DATE: 26-AUG-1993
   (viii) ATTORNEY/AGENT INFORMATION:
      (A) NAME: McDaniels, Patricia A.
      (B) REGISTRATION NUMBER: 33,194
      (C) REFERENCE/DOCKET NUMBER: GI 5213B-PCT
   (ix) TELECOMMUNICATION INFORMATION:
      (A) TELEPHONE: (617) 876-1210 Ext. 8405
      (B) TELEFAX: (617) 876-5851
(2) INFORMATION FOR SEQ ID NO:1:
   (i) SEQUENCE CHARACTERISTICS:
      (A) LENGTH: 1649 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
      (G) CELL TYPE: Promyelocyte
      (H) CELL LINE: HL60
   (vii) IMMEDIATE SOURCE:
      (B) CLONE: pMT21:PL85
   (ix) FEATURE:
      (A) NAME/KEY: 5'UTR
      (B) LOCATION: 1..59
   (ix) FEATURE:
      (A) NAME/KEY: CDS
      (B) LOCATION: 60..1268
   (ix) FEATURE:
      (A) NAME/KEY: 3'UTR
      (B) LOCATION: 1269..1649
   (xi) SEQUENCE DESCRIPTION: SEQ ID NO:1:
   GCCACTTCTT CTGGGCCCAC GAGGCAGCTG TCCCATGCTC TGCTGAGCAC GGTGGTGCC        59
   ATG CCT CTG CAA CTC CTC CTG TTG CTG ATC CTA CTG GGC CCT GGC AAC        107
   Met Pro Leu Gln Leu Leu Leu Leu Leu Ile Leu Leu Gly Pro Gly Asn
     1               5                  10                  15
   AGC TTG CAG CTG TGG GAC ACC TGG GCA GAT GAA GCC GAG AAA GCC TTG        155
   Ser Leu Gln Leu Trp Asp Thr Trp Ala Asp Glu Ala Glu Lys Ala Leu
                20                  25                  30
   GGT CCC CTG CTT GCC CGG GAC CGG AGA CAG GCC ACC GAA TAT GAG TAC        203
   Gly Pro Leu Leu Ala Arg Asp Arg Arg Gln Ala Thr Glu Tyr Glu Tyr
            35                  40                  45
   CTA GAT TAT GAT TTC CTG CCA GAA ACG GAG CCT CCA GAA ATG CTG AGG        251
   Leu Asp Tyr Asp Phe Leu Pro Glu Thr Glu Pro Pro Glu Met Leu Arg
        50                  55                  60
   AAC AGC ACT GAC ACC ACT CCT CTG ACT GGG CCT GGA ACC CCT GAG TCT        299
   Asn Ser Thr Asp Thr Thr Pro Leu Thr Gly Pro Gly Thr Pro Glu Ser
    65                  70                  75                  80
   ACC ACT GTG GAG CCT GCT GCA AGG CGT TCT ACT GGC CTG GAT GCA GGA        347
   Thr Thr Val Glu Pro Ala Ala Arg Arg Ser Thr Gly Leu Asp Ala Gly
                    85                  90                  95
   GGG GCA GTC ACA GAG CTG ACC ACG GAG CTG GCC AAC ATG GGG AAC CTG        395
   Gly Ala Val Thr Glu Leu Thr Thr Glu Leu Ala Asn Met Gly Asn Leu
               100                 105                 110
   TCC ACG GAT TCA GCA GCT ATG GAG ATA CAG ACC ACT CAA CCA GCA GCC        443
   Ser Thr Asp Ser Ala Ala Met Glu Ile Gln Thr Thr Gln Pro Ala Ala
           115                 120                 125
   ACG GAG GCA CAG ACC ACT CCA CTG GCA GCC ACA GAG GCA CAG ACA ACT        491
   Thr Glu Ala Gln Thr Thr Pro Leu Ala Ala Thr Glu Ala Gln Thr Thr
       130                 135                 140
   CGA CTG ACG GCC ACG GAG GCA CAG ACC ACT CCA CTG GCA GCC ACA GAG      539
   Arg Leu Thr Ala Thr Glu Ala Gln Thr Thr Pro Leu Ala Ala Thr Glu
   145                 150                 155                 160
   GCA CAG ACC ACT CCA CCA GCA GCC ACG GAA GCA CAG ACC ACT CAA CCC      587
   Ala Gln Thr Thr Pro Pro Ala Ala Thr Glu Ala Gln Thr Thr Gln Pro
                   165                 170                 175
   ACA GGC CTG GAG GCA CAG ACC ACT GCA CCA GCA GCC ATG GAG GCA CAG      635
   Thr Gly Leu Glu Ala Gln Thr Thr Ala Pro Ala Ala Met Glu Ala Gln
               180                 185                 190
   ACC ACT GCA CCA GCA GCC ATG GAA GCA CAG ACC ACT CCA CCA GCA GCC      683
   Thr Thr Ala Pro Ala Ala Met Glu Ala Gln Thr Thr Pro Pro Ala Ala
           195                 200                 205
   ATG GAG GCA CAG ACC ACT CAA ACC ACA GCC ATG GAG GCA CAG ACC ACT      731
   Met Glu Ala Gln Thr Thr Gln Thr Thr Ala Met Glu Ala Gln Thr Thr
       210                 215                 220
   GCA CCA GAA GCC ACG GAG GCA CAG ACC ACT CAA CCC ACA GCC ACG GAG      779
   Ala Pro Glu Ala Thr Glu Ala Gln Thr Thr Gln Pro Thr Ala Thr Glu
   225                 230                 235                 240
   GCA CAG ACC ACT CCA CTG GCA GCC ATG GAG GCC CTG TCC ACA GAA CCC      827
   Ala Gln Thr Thr Pro Leu Ala Ala Met Glu Ala Leu Ser Thr Glu Pro
                   245                 250                 255
   AGT GCC ACA GAG GCC CTG TCC ATG GAA CCT ACT ACC AAA AGA GGT CTG      875
   Ser Ala Thr Glu Ala Leu Ser Met Glu Pro Thr Thr Lys Arg Gly Leu
               260                 265                 270
   TTC ATA CCC TTT TCT GTG TCC TCT GTT ACT CAC AAG GGC ATT CCC ATG      923
   Phe Ile Pro Phe Ser Val Ser Ser Val Thr His Lys Gly Ile Pro Met
           275                 280                 285
   GCA GCC AGC AAT TTG TCC GTC AAC TAC CCA GTG GGG GCC CCA GAC CAC      971
   Ala Ala Ser Asn Leu Ser Val Asn Tyr Pro Val Gly Ala Pro Asp His
       290                 295                 300
   ATC TCT GTG AAG CAG TGC CTG CTG GCC ATC CTA ATC TTG GCG CTG GTG     1019
   Ile Ser Val Lys Gln Cys Leu Leu Ala Ile Leu Ile Leu Ala Leu Val
   305                 310                 315                 320
   GCC ACT ATC TTC TTC GTG TGC ACT GTG GTG CTG GCG GTC CGC CTC TCC     1067
   Ala Thr Ile Phe Phe Val Cys Thr Val Val Leu Ala Val Arg Leu Ser
                   325                 330                 335
   CGC AAG GGC CAC ATG TAC CCC GTG CGT AAT TAC TCC CCC ACC GAG ATG     1115
   Arg Lys Gly His Met Tyr Pro Val Arg Asn Tyr Ser Pro Thr Glu Met
               340                 345                 350
   GTC TGC ATC TCA TCC CTG TTG CCT GAT GGG GGT GAG GGG CCC TCT GCC     1163
   Val Cys Ile Ser Ser Leu Leu Pro Asp Gly Gly Glu Gly Pro Ser Ala
           355                 360                 365
   ACA GCC AAT GGG GGC CTG TCC AAG GCC AAG AGC CCG GGC CTG ACG CCA     1211
   Thr Ala Asn Gly Gly Leu Ser Lys Ala Lys Ser Pro Gly Leu Thr Pro
       370                 375                 380
   GAG CCC AGG GAG GAC CGT GAG GGG GAT GAC CTC ACC CTG CAC AGC TTC     1259
   Glu Pro Arg Glu Asp Arg Glu Gly Asp Asp Leu Thr Leu His Ser Phe
   385                 390                 395                 400
   CTC CCT TAGCTCACTC TGCCATCTGT TTTGGCAAGA CCCCACCTCC ACGGGCTCTC      1315
   Leu Pro
   CTGGGCCACC CCTGAGTGCC CAGACCCCAA TCCACAGCTC TGGGCTTCCT CGGAGACCCC 1375
   TGGGGATGGG GATCTTCAGG GAAGGAACTC TGGCCACCCA AACAGGACAA GAGCAGCCTG 1435
   GGGCCAAGCA GACGGGCAAG TGGAGCCACC TCTTTCCTCC CTCCGCGGAT GAAGCCCAGC 1495
   CACATTTCAG CCGAGGTCCA AGGCAGGAGG CCATTTACTT GAGACAGATT CTCTCCTTTT 1555
   TCCTGTCCCC CATCTTCTCT GGGTCCCTCT AACATCTCCC ATGGCTCTCC CCGCTTCTCC 1615
   TGGTCACTGG AGTCTCCTCC CCATGTACCC AAGG 1649
(2) INFORMATION FOR SEQ ID NO:2:
   (i) SEQUENCE CHARACTERISTICS:
      (A) LENGTH: 402 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Pro Leu Gln Leu Leu Leu Leu Leu Ile Leu Leu Gly Pro Gly Asn
   1 5 10 15
   Ser Leu Gln Leu Trp Asp Thr Trp Ala Asp Glu Ala Glu Lys Ala Leu
   20 25 30
   Gly Pro Leu Leu Ala Arg Asp Arg Arg Gln Ala Thr Glu Tyr Glu Tyr
   35 40 45
   Leu Asp Tyr Asp Phe Leu Pro Glu Thr Glu Pro Pro Glu Met Leu Arg
   50 55 60
   Asn Ser Thr Asp Thr Thr Pro Leu Thr Gly Pro Gly Thr Pro Glu Ser
   65 70 75 80
   Thr Thr Val Glu Pro Ala Ala Arg Arg Ser Thr Gly Leu Asp Ala Gly
   85 90 95
   Gly Ala Val Thr Glu Leu Thr Thr Glu Leu Ala Asn Met Gly Asn Leu
   100 105 110
   Ser Thr Asp Ser Ala Ala Met Glu Ile Gln Thr Thr Gln Pro Ala Ala
   115 120 125
   Thr Glu Ala Gln Thr Thr Pro Leu Ala Ala Thr Glu Ala Gln Thr Thr
   130 135 140
   Arg Leu Thr Ala Thr Glu Ala Gln Thr Thr Pro Leu Ala Ala Thr Glu
   145 150 155 160
   Ala Gln Thr Thr Pro Pro Ala Ala Thr Glu Ala Gln Thr Thr Gln Pro
   165 170 175
   Thr Gly Leu Glu Ala Gln Thr Thr Ala Pro Ala Ala Met Glu Ala Gln
   180 185 190
   Thr Thr Ala Pro Ala Ala Met Glu Ala Gln Thr Thr Pro Pro Ala Ala
   195 200 205
   Met Glu Ala Gln Thr Thr Gln Thr Thr Ala Met Glu Ala Gln Thr Thr
   210 215 220
   Ala Pro Glu Ala Thr Glu Ala Gln Thr Thr Gln Pro Thr Ala Thr Glu
   225 230 235 240
   Ala Gln Thr Thr Pro Leu Ala Ala Met Glu Ala Leu Ser Thr Glu Pro
   245 250 255
   Ser Ala Thr Glu Ala Leu Ser Met Glu Pro Thr Thr Lys Arg Gly Leu
   260 265 270
   Phe Ile Pro Phe Ser Val Ser Ser Val Thr His Lys Gly Ile Pro Met
   275 280 285
   Ala Ala Ser Asn Leu Ser Val Asn Tyr Pro Val Gly Ala Pro Asp His
   290 295 300
   Ile Ser Val Lys Gln Cys Leu Leu Ala Ile Leu Ile Leu Ala Leu Val
   305 310 315 320
   Ala Thr Ile Phe Phe Val Cys Thr Val Val Leu Ala Val Arg Leu Ser
   325 330 335
   Arg Lys Gly His Met Tyr Pro Val Arg Asn Tyr Ser Pro Thr Glu Met
   340 345 350
   Val Cys Ile Ser Ser Leu Leu Pro Asp Gly Gly Glu Gly Pro Ser Ala
   355 360 365
   Thr Ala Asn Gly Gly Leu Ser Lys Ala Lys Ser Pro Gly Leu Thr Pro
   370 375 380
   Glu Pro Arg Glu Asp Arg Glu Gly Asp Asp Leu Thr Leu His Ser Phe
   385 390 395 400
   Leu Pro
(2) INFORMATION FOR SEQ ID NO:3:
   (i) SEQUENCE CHARACTERISTICS:
      (A) LENGTH: 1239 base pairs
      (B) TYPE: nucleic acid
      (C) STRANDEDNESS: single
      (D) TOPOLOGY: linear
      (ii) MOLECULE TYPE: cDNA (synthetic)
   (vi) ORIGINAL SOURCE:
      (A) ORGANISM: Homo sapiens
      (G) CELL TYPE: placenta
   (ix) FEATURE:
      (A) NAME/KEY: CDS
      (B) LOCATION: 1..1239
   (xi) SEQUENCE DESCRIPTION: SEQ ID NO:3:
   ATG CCT CTG CAA CTC CTC CTG TTG CTG ATC CTA CTG GGC CCT GGC AAC    48
   Met Pro Leu Gln Leu Leu Leu Leu Leu Ile Leu Leu Gly Pro Gly Asn
   1               5                   10                  15
   AGC TTG CAG CTG TGG GAC ACC TGG GCA GAT GAA GCC GAG AAA GCC TTG    96
   Ser Leu Gln Leu Trp Asp Thr Trp Ala Asp Glu Ala Glu Lys Ala Leu
               20                  25                  30
   GGT CCC CTG CTT GCC CGG GAC CGG AGA CAG GCC ACC GAA TAT GAG TAC   144
   Gly Pro Leu Leu Ala Arg Asp Arg Arg Gln Ala Thr Glu Tyr Glu Tyr
           35                  40                  45
   CTA GAT TAT GAT TTC CTG CCA GAA ACG GAG CCT CCA GAA ATG CTG AGG   192
   Leu Asp Tyr Asp Phe Leu Pro Glu Thr Glu Pro Pro Glu Met Leu Arg
       50                  55                  60
   AAC AGC ACT GAC ACC ACT CCT CTG ACT GGG CCT GGA ACC CCT GAG TCT   240
   Asn Ser Thr Asp Thr Thr Pro Leu Thr Gly Pro Gly Thr Pro Glu Ser
   65                  70                  75                  80
   ACC ACT GTG GAG CCT GCT GCA AGG CGT TCT ACT GGC CTG GAT GCA GGA   288
   Thr Thr Val Glu Pro Ala Ala Arg Arg Ser Thr Gly Leu Asp Ala Gly
                   85                  90                  95
   GGG GCA GTC ACA GAG CTG ACC ACG GAG CTG GCC AAC ATG GGG AAC CTG        336
   Gly Ala Val Thr Glu Leu Thr Thr Glu Leu Ala Asn Met Gly Asn Leu
               100                 105                 110
   TCC ACG GAT TCA GCA GCT ATG GAG ATA CAG ACC ACT CAA CCA GCA GCC        384
   Ser Thr Asp Ser Ala Ala Met Glu Ile Gln Thr Thr Gln Pro Ala Ala
           115                 120                 125
   ACG GAG GCA CAG ACC ACT CAA CCA GTG CCC ACG GAG GCA CAG ACC ACT        432
   Thr Glu Ala Gln Thr Thr Gln Pro Val Pro Thr Glu Ala Gln Thr Thr
       130                 135                 140
   CCA CTG GCA GCC ACA GAG GCA CAG ACA ACT CGA CTG ACG GCC ACG GAG        480
   Pro Leu Ala Ala Thr Glu Ala Gln Thr Thr Arg Leu Thr Ala Thr Glu
   145                 150                 155                 160
   GCA CAG ACC ACT CCA CTG GCA GCC ACA GAG GCA CAG ACC ACT CCA CCA        528
   Ala Gln Thr Thr Pro Leu Ala Ala Thr Glu Ala Gln Thr Thr Pro Pro
                   165                 170                 175
   GCA GCC ACG GAA GCA CAG ACC ACT CAA CCC ACA GGC CTG GAG GCA CAG        576
   Ala Ala Thr Glu Ala Gln Thr Thr Gln Pro Thr Gly Leu Glu Ala Gln
               180                 185                 190
   ACC ACT GCA CCA GCA GCC ATG GAG GCA CAG ACC ACT GCA CCA GCA GCC        624
   Thr Thr Ala Pro Ala Ala Met Glu Ala Gln Thr Thr Ala Pro Ala Ala
           195                 200                 205
   ATG GAA GCA CAG ACC ACT CCA CCA GCA GCC ATG GAG GCA CAG ACC ACT        672
   Met Glu Ala Gln Thr Thr Pro Pro Ala Ala Met Glu Ala Gln Thr Thr
       210                 215                 220
   CAA ACC ACA GCC ATG GAG GCA CAG ACC ACT GCA CCA GAA GCC ACG GAG        720
   Gln Thr Thr Ala Met Glu Ala Gln Thr Thr Ala Pro Glu Ala Thr Glu
   225                 230                 235                 240
   GCA CAG ACC ACT CAA CCC ACA GCC ACG GAG GCA CAG ACC ACT CCA CTG        768
   Ala Gln Thr Thr Gln Pro Thr Ala Thr Glu Ala Gln Thr Thr Pro Leu
                   245                 250                 255
   GCA GCC ATG GAG GCC CTG TCC ACA GAA CCC AGT GCC ACA GAG GCC CTG        816
   Ala Ala Met Glu Ala Leu Ser Thr Glu Pro Ser Ala Thr Glu Ala Leu
               260                 265                 270
   TCC ATG GAA CCT ACT ACC AAA AGA GGT CTG TTC ATA CCC TTT TCT GTG        864
   Ser Met Glu Pro Thr Thr Lys Arg Gly Leu Phe Ile Pro Phe Ser Val
           275                 280                 285
   TCC TCT GTT ACT CAC AAG GGC ATT CCC ATG GCA GCC AGC AAT TTG TCC        912
   Ser Ser Val Thr His Lys Gly Ile Pro Met Ala Ala Ser Asn Leu Ser
       290                 295                 300
   GTC AAC TAC CCA GTG GGG GCC CCA GAC CAC ATC TCT GTG AAG CAG TGC        960
   Val Asn Tyr Pro Val Gly Ala Pro Asp His Ile Ser Val Lys Gln Cys
   305                 310                 315                 320
   CTG CTG GCC ATC CTA ATC TTG GCG CTG GTG GCC ACT ATC TTC TTC GTG       1008
   Leu Leu Ala Ile Leu Ile Leu Ala Leu Val Ala Thr Ile Phe Phe Val
                   325                 330                 335
   TGC ACT GTG GTG CTG GCG GTC CGC CTC TCC CGC AAG GGC CAC ATG TAC       1056
   Cys Thr Val Val Leu Ala Val Arg Leu Ser Arg Lys Gly His Met Tyr
               340                 345                 350
   CCC GTG CGT AAT TAC TCC CCC ACC GAG ATG GTC TGC ATC TCA TCC CTG       1104
   Pro Val Arg Asn Tyr Ser Pro Thr Glu Met Val Cys Ile Ser Ser Leu
           355                 360                 365
   TTG CCT GAT GGG GGT GAG GGG CCC TCT GCC ACA GCC AAT GGG GGC CTG        1152
   Leu Pro Asp Gly Gly Glu Gly Pro Ser Ala Thr Ala Asn Gly Gly Leu
       370                 375                 380
   TCC AAG GCC AAG AGC CCG GGC CTG ACG CCA GAG CCC AGG GAG GAC CGT        1200
   Ser Lys Ala Lys Ser Pro Gly Leu Thr Pro Glu Pro Arg Glu Asp Arg
   385                 390                 395                 400
   GAG GGG GAT GAC CTC ACC CTG CAC AGC TTC CTC CCT TAG                    1239
   Glu Gly Asp Asp Leu Thr Leu His Ser Phe Leu Pro
                   405                 410
(2) INFORMATION FOR SEQ ID NO:4:
   (i) SEQUENCE CHARACTERISTICS:
      (A) LENGTH: 412 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Pro Leu Gln Leu Leu Leu Leu Leu Ile Leu Leu Gly Pro Gly Asn
   1               5                   10                  15
   Ser Leu Gln Leu Trp Asp Thr Trp Ala Asp Glu Ala Glu Lys Ala Leu
               20                  25                  30
   Gly Pro Leu Leu Ala Arg Asp Arg Arg Gln Ala Thr Glu Tyr Glu Tyr
           35                  40                  45
   Leu Asp Tyr Asp Phe Leu Pro Glu Thr Glu Pro Pro Glu Met Leu Arg
       50                  55                  60
   Asn Ser Thr Asp Thr Thr Pro Leu Thr Gly Pro Gly Thr Pro Glu Ser
   65                  70                  75                  80
   Thr Thr Val Glu Pro Ala Ala Arg Arg Ser Thr Gly Leu Asp Ala Gly
                   85                  90                  95
   Gly Ala Val Thr Glu Leu Thr Thr Glu Leu Ala Asn Met Gly Asn Leu
               100                 105                 110
   Ser Thr Asp Ser Ala Ala Met Glu Ile Gln Thr Thr Gln Pro Ala Ala
           115                 120                 125
   Thr Glu Ala Gln Thr Thr Gln Pro Val Pro Thr Glu Ala Gln Thr Thr
   130 135 140
   Pro Leu Ala Ala Thr Glu Ala Gln Thr Thr Arg Leu Thr Ala Thr Glu
   145 150 155 160
   Ala Gln Thr Thr Pro Leu Ala Ala Thr Glu Ala Gln Thr Thr Pro Pro
   165 170 175
   Ala Ala Thr Glu Ala Gln Thr Thr Gln Pro Thr Gly Leu Glu Ala Gln
   180 185 190
   Thr Thr Ala Pro Ala Ala Met Glu Ala Gln Thr Thr Ala Pro Ala Ala
   195 200 205
   Met Glu Ala Gln Thr Thr Pro Pro Ala Ala Met Glu Ala Gln Thr Thr
   210 215 220
   Gln Thr Thr Ala Met Glu Ala Gln Thr Thr Ala Pro Glu Ala Thr Glu
   225 230 235 240
   Ala Gln Thr Thr Gln Pro Thr Ala Thr Glu Ala Gln Thr Thr Pro Leu
   245 250 255
   Ala Ala Met Glu Ala Leu Ser Thr Glu Pro Ser Ala Thr Glu Ala Leu
   260 265 270
   Ser Met Glu Pro Thr Thr Lys Arg Gly Leu Phe Ile Pro Phe Ser Val
   275 280 285
   Ser Ser Val Thr His Lys Gly Ile Pro Met Ala Ala Ser Asn Leu Ser
   290 295 300
   Val Asn Tyr Pro Val Gly Ala Pro Asp His Ile Ser Val Lys Gln Cys
   305 310 315 320
   Leu Leu Ala Ile Leu Ile Leu Ala Leu Val Ala Thr Ile Phe Phe Val
   325 330 335
   Cys Thr Val Val Leu Ala Val Arg Leu Ser Arg Lys Gly His Met Tyr
   340 345 350
   Pro Val Arg Asn Tyr Ser Pro Thr Glu Met Val Cys Ile Ser Ser Leu
   355 360 365
   Leu Pro Asp Gly Gly Glu Gly Pro Ser Ala Thr Ala Asn Gly Gly Leu
   370 375 380
   Ser Lys Ala Lys Ser Pro Gly Leu Thr Pro Glu Pro Arg Glu Asp Arg
   385 390 395 400
   Glu Gly Asp Asp Leu Thr Leu His Ser Phe Leu Pro
   405 410
(2) INFORMATION FOR SEQ ID NO:5:
   (i) SEQUENCE CHARACTERISTICS:
      (A) LENGTH: 2151 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: pacesol
   (xi) SEQUENCE DESCRIPTION: SEQ ID NO:5:
   ATGGAGCTGA GGCCCTGGTT GCTATGGGTG GTAGCAGCAA CAGGAACCTT GGTCCTGCTA    60
   GCAGCTGATG CTCAGGGCCA GAAGGTCTTC ACCAACACGT GGGCTGTGCG CATCCCTGGA   120
   GGCCCAGCGG TGGCCAACAG TGTGGCACGG AAGCATGGGT TCCTCAACCT GGGCCAGATC   180
   TTCGGGGACT ATTACCACTT CTGGCATCGA GGAGTGACGA AGCGGTCCCT GTCGCCTCAC   240
   CGCCCGCGGC ACAGCCGGCT GCAGAGGGAG CCTCAAGTAC AGTGGCTGGA ACAGCAGGTG   300
   GCAAAGCGAC GGACTAAACG GGACGTGTAC CAGGAGCCCA CAGACCCCAA GTTTCCTCAG   360
   CAGTGGTACC TGTCTGGTGT CACTCAGCGG GACCTGAATG TGAAGGCGGC CTGGGCGCAG   420
   GGCTACACAG GGCACGGCAT TGTGGTCTCC ATTCTGGACG ATGGCATCGA GAAGAACCAC   480
   CCGGACTTGG CAGGCAATTA TGATCCTGGG GCCAGTTTTG ATGTCAATGA CCAGGACCCT   540
   GACCCCCAGC CTCGGTACAC ACAGATGAAT GACAACAGGC ACGGCACACG GTGTGCGGGG   600
   GAAGTGGCTG CGGTGGCCAA CAACGGTGTC TGTGGTGTAG GTGTGGCCTA CAACGCCCGC   660
   ATTGGAGGGG TGCGCATGCT GGATGGCGAG GTGACAGATG CAGTGGAGGC ACGCTCGCTG   720
   GGCCTGAACC CCAACCACAT CCACATCTAC AGTGCCAGCT GGGGCCCCGA GGATGACGGC   780
   AAGACAGTGG ATGGGCCAGC CCGCCTCGCC GAGGAGGCCT TCTTCCGTGG GGTTAGCCAG   840
   GGCCGAGGGG GGCTGGGCTC CATCTTTGTC TGGGCCTCGG GGAACGGGGG CCGGGAACAT   900
   GACAGCTGCA ACTGCGACGG CTACACCAAC AGTATCTACA CGCTGTCCAT CAGCAGCGCC   960
   ACGCAGTTTG GCAACGTGCC GTGGTACAGC GAGGCCTGCT CGTCCACACT GGCCACGACC  1020
   TACAGCAGTG GCAACCAGAA TGAGAAGCAG ATCGTGACGA CTGACTTGCG GCAGAAGTGC    1080
   ACGGAGTCTC ACACGGGCAC CTCAGCCTCT GCCCCCTTAG CAGCCGGCAT CATTGCTCTC    1140
   ACCCTGGAGG CCAATAAGAA CCTCACATGG CGGGACATGC AACACCTGGT GGTACAGACC    1200
   TCGAAGCCAG CCCACCTCAA TGCCAACGAC TGGGCCACCA ATGGTGTGGG CCGGAAAGTG    1260
   AGCCACTCAT ATGGCTACGG GCTTTTGGAC GCAGGCGCCA TGGTGGCCCT GGCCCAGAAT    1320
   TGGACCACAG TGGCCCCCCA GCGGAAGTGC ATCATCGACA TCCTCACCGA GCCCAAAGAC    1380
   ATCGGGAAAC GGCTCGAGGT GCGGAAGACC GTGACCGCGT GCCTGGGCGA GCCCAACCAC    1440
   ATCACTCGGC TGGAGCACGC TCAGGCGCGG CTCACCCTGT CCTATAATCG CCGTGGCGAC    1500
   CTGGCCATCC ACCTGGTCAG CCCCATGGGC ACCCGCTCCA CCCTGCTGGC AGCCAGGCCA    1560
   CATGACTACT CCGCAGATGG GTTTAATGAC TGGGCCTTCA TGACAACTCA TTCCTGGGAT    1620
   GAGGATCCCT CTGGCGAGTG GGTCCTAGAG ATTGAAAACA CCAGCGAAGC CAACAACTAT    1680
   GGGACGCTGA CCAAGTTCAC CCTCGTACTC TATGGCACCG CCCCTGAGGG GCTGCCCGTA    1740
   CCTCCAGAAA GCAGTGGCTG CAAGACCCTC ACGTCCAGTC AGGCCTGTGT GGTGTGCGAG    1800
   GAAGGCTTCT CCCTGCACCA GAAGAGCTGT GTCCAGCACT GCCCTCCAGG CTTCGCCCCC    1860
   CAAGTCCTCG ATACGCACTA TAGCACCGAG AATGACGTGG AGACCATCCG GGCCAGCGTC    1920
   TGCGCCCCCT GCCACGCCTC ATGTGCCACA TGCCAGGGGC CGGCCCTGAC AGACTGCCTC    1980
   AGCTGCCCCA GCCACGCCTC CTTGGACCCT GTGGAGCAGA CTTGCTCCCG GCAAAGCCAG    2040
   AGCAGCCGAG AGTCCCCGCC ACAGCAGCAG CCACCTCGGC TGCCCCCGGA GGTGGAGGCG    2100
   GGGCAACGGC TGCGGGCAGG GCTGCTGCCC TCACACCTGC CTGAGTGATG A             2151
(2) INFORMATION FOR SEQ ID N0:6:
   (i) SEQUENCE CHARACTERISTICS:
      (A) LENGTH: 1591 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (vii) IMMEDIATE SOURCE:
      (B) CLONE: sPSL.Fc
   (xi) SEQUENCE DESCRIPTION: SEQ ID NO:6: